# EUROPEAN PATENT SPECIFICATION

(11) **EP 1 877 397 B1**
(45) Date of publication and mention of the grant of the patent: **05.08.2009**
(21) Application number: 06726940.7
(22) Date of filing: 27.04.2006
(51) Int. Cl.: C07D 401/14, A61K 31/517, A61P 35/00

(54) **QUINAZOLINE DERIVATIVES AS INHIBITORS OF EGF AND/OR ERBB2 RECEPTOR TYROSINE KINASE**
CHINAZOLINDERIVATE ALS INHIBITOREN VON EGF UND/ODER ERBB2-REZEPTORTYROSINKINASE
DERIVES DE QUINAZOLINE COMME INHIBITEURS DE EGF ET/OU DU RECEPTEUR ERBB2 DE LA TYROSINE KINASE

(30) Priority: 29.04.2005 GB 0508717
(43) Date of publication of application: 16.01.2008
(73) Proprietor: AstraZeneca AB, 151 85 Södertälje (SE)
(72) Inventor: BRADBURY, Robert Hugh, Macclesfield Cheshire SK10 4TG (GB)
(74) Representative: Nelson, Michael Andrew
(86) International application number: PCT/GB2006/001560
(87) International publication number: WO 2006/117521

(56) References cited:
- WO-A-03/040108
- WO-A-03/040109
- WO-A-20/04093880
- WO-A-20/05051923

## Description

The invention concerns certain novel quinazoline derivatives, or pharmaceutically acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors and other autocrine, paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised into 20 receptor tyrosine kinase and 10 non-receptor tyrosine kinase sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that resides in the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000,77,25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res.,1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga et al., Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res.; 2001, 7, 1850; Ross et al, Cancer Investigation, 2001, 19, 554, Yu et al., Bioessays, 2000, 22.7, 673).

In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565).

In addition to this pre-clinical data, the small molecule EGFR tyrosine kinase inhibitors Iressa® (also known as gefitinib and ZD1839) and Tarceva® (also known as erlotinib and CP-358,774) have been approved for use in the treatment of advanced non-small cell lung cancer. Furthermore, inhibitory antibodies against EGFR and erbB2 (erbitux® (c-225 / cetuximab) and herceptin® (trastuzumab) respectively) have proven to be beneficial in the clinic for the treatment of selected solid tumours (reviewed in Mendelsohn et al, 2000, Oncogene, 19, 6550-6565).

Recently mutations in the ATP binding pocket of the intracellular catalytic domain of the EGF receptor have been discovered in certain sub-sets of non-small cell lung cancers (NSCLCs). The presence of mutations in the receptor appear to correlate with response to EGFR tyrosine kinase inhibitors such as gefitinib (Lynch et al, N Engl J Med 2004; 350: 2129-2139; Paez et al, Science 2004; 304:1497-1500), although it is becoming evident that the clinical benefits of compounds such as gefitinib and erlotinib are not likely to be mediated by EGFR mutations alone. It has been demonstrated that ligand stimulation results in a different phosphorylation pattern in mutated receptors compared with that seen in wild-type receptors and it is thought that mutant EGF receptors selectively transduce survival signals on which NSCLCs become dependent. Inhibition of those signals by compounds such as gefitinib may contribute to the efficacy of such drugs (Sordella et al. Science 2004; 305: 1163-1167). Similarly, mutations within the erbB2 kinase domain have recently been discovered in certain primary tumours, such as NSCLC, glioblastoma and gastric and ovarian tumours (Stephens et al., Nature 2004; 431; 525-526). Accordingly the inhibition of the EGF and/or erbB2 tyrosine kinase in both wild-type and mutated receptors is an important target that would be expected to provide an anti-cancer effect.

Amplification and/or activity of members of the erbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

WO 96/09294, WO 96/15118, WO 96/16960, WO 96/30347, WO 96/33977, WO 96/33978, WO 96/33979, WO 96/33980, WO 96/33981, WO 97/03069, WO 97/13771, WO 97/30034, WO 97/30035, WO 97/38983, WO 98/02437, WO 98/02434, WO 98/02438, WO 98/13354, WO 99/35132, WO 99/35146, WO 01/21596, WO 01/55141 and WO 02/18372 each disclose that certain quinazoline derivatives which bear an anilino substituent at the 4-position possess receptor tyrosine kinase inhibitory activity.

WO 01/94341 discloses that certain quinazoline derivatives which carry a 5-substituent are inhibitors of the Src family of non-receptor tyrosine kinases, such as c-Src, c-Yes and c-Fyn. There is no disclosure in WO 01/94341 of 4-(indol-5-ylamino)quinazoline derivatives wherein the nitrogen atom of the indolyl group is substituted by a substituent containing an aryl or a heteroaryl group.

WO 03/040108 and WO 03/040109 each disclose that certain quinazoline derivatives which carry a 5-substituent are inhibitors of the erbB family of tyrosine kinase inhibitors, particularly EGF and erbB2 receptor tyrosine kinases. WO 03/040108 and WO 03/040109 each disclose certain 4-(indol-5-ylamino)quinazoline derivatives. None of the quinazoline derivatives disclosed contain an acylaminoethoxy group at the 5-position on the quinazoline ring.

WO 02/034744 discloses certain 4-(indol-7-ylamino)quinazoline derivatives and their use in containing and/or treating solid tumour disease, for example by inhibition against the Src family of non-receptor tyrosine kinase. The quinazoline derivatives disclosed do not contain a substituent at the 5-position on the quinazoline ring or an (indol-5-ylamino) substituent at the 4-position on the quinazoline ring.

US-2004/0048880 discloses certain 4-anilinoquinazoline derivatives and their use in treating tumoural diseases. The quinazoline derivatives disclosed do not contain a substituent at the 5-position on the quinazoline ring.

WO 2004/46101 discloses certain quinazoline derivatives and their use as inhibitors of EGF and erbB2 receptor tyrosine kinases. The quinazoline derivatives disclosed do not contain a substituent at the 5-position on the quinazoline ring. Additionally, there is no disclosure of a 4-(indol-5-ylamino)quinazoline derivative.

WO 2004/093880 and WO 2005/051923 each disclose certain 4-anilinoquinazoline derivatives and their use as inhibitors of erbB2 receptor tyrosine kinase. Neither of these documents discloses a 4-(indol-5-ylamino)quinazoline derivative.

There remains a need to find further compounds with good *in-vivo* activity together with improved pharmacological characteristics compared with known erbB tyrosine kinase inhibitors, particularly compounds that are selective erbB2 tyrosine kinase inhibitors. For example, there is a need for novel compounds with advantageous and/or improved characteristics in, but not limited to, for example, (i) physical properties; (ii) favourable DMPK properties, such as high bioavailability and/ or advantageous half life and/or advantageous volume of distribution and/or high absorption; (iii) factors that decrease the liability for clinical drug-drug interactions (e.g. cytochrome P450 enzyme inhibition or induction); and (iv) compounds with a reduced liability for QT interval prolongation in patients, for example compounds which are inactive or weakly active in a HERG assay.

Surprisingly, we have now found that a select group of 4-(indol-5-ylamino)quinazoline derivatives substituted at the 5-position with a substituent containing certain acylaminoethoxy groups possess potent anti-tumour activity. Without wishing to imply that the quinazoline derivatives disclosed in the present invention possess pharmacological activity only by virtue of an effect on a single biological process, it is believed that the quinazoline derivatives provide an anti-tumour effect by way of inhibition of one or more of the erbB family of receptor tyrosine kinases that are involved in the signal transduction steps which lead to the proliferation of tumour cells. In particular, it is believed that the quinazoline derivatives of the present invention provide an anti-tumour effect by way of inhibition of EGF and/or erbB2 receptor tyrosine kinases. More particularly, it is believed that the quinazoline derivatives of the present invention provide an anti-tumour effect by way of the selective inhibition of erbB2 receptor tyrosine kinase, compared to EGF receptor tyrosine kinase. It is also believed that the quinazoline derivatives of the present invention exhibit a combination of favourable properties, such as those described hereinbefore.

References to erbB receptors, particularly erbB2, used herein are intended to include both wild-type and mutated receptors unless specifically stated otherwise. The term "mutation" includes, but is not limited to, gene amplification, nucleotide in-frame deletions or substitutions in one or more of the exons that encode receptors such as erbB2.

Generally the quinazoline derivatives of the present invention possess potent inhibitory activity against the erbB receptor tyrosine kinase family, for example by inhibition of EGF and/or erbB2 and/or erbB4 receptor tyrosine kinases, whilst possessing less potent inhibitory activity against other kinases. Furthermore, generally the quinazoline derivatives of the present invention possess substantially better potency against the erbB2 tyrosine kinase over that of the EGFR tyrosine kinase, thus potentially providing effective treatment for erbB2 driven tumours. Accordingly, it may be possible to administer a quinazoline derivative according to the present invention at a dose that is sufficient to inhibit erbB2 tyrosine kinase whilst having no significant effect upon EGFR or other tyrosine kinases. The selective inhibition provided by the quinazoline derivatives according to the present invention may provide treatments for conditions mediated by erbB2 tyrosine kinase, whilst reducing undesirable side effects that may be associated with the inhibition of other tyrosine kinases.

According to a first aspect of the invention there is provided a quinazoline derivative of the Formula **I**: 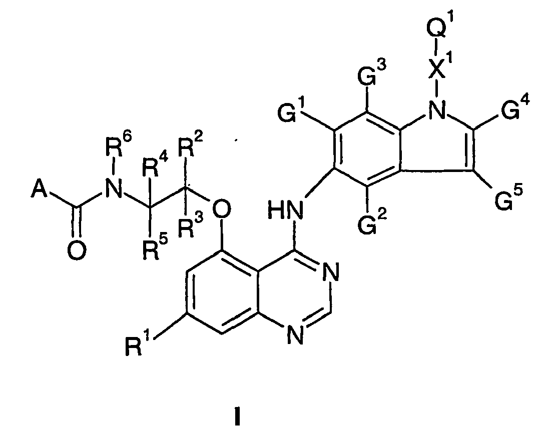 wherein:
**R¹** is selected from hydrogen, hydroxy, (1-4C)alkoxy and (1-4C)alkoxy(1-4C)alkoxy;
**G¹, G², G³, G⁴ and G⁵** are each, independently, selected from hydrogen and halogeno;
**X¹** is selected from SO₂, CO, SO₂N(R⁷) and C(R⁷)₂, wherein each R⁷ is, independently, selected from hydrogen and (1-4C)alkyl;
**Q¹** is aryl or heteroaryl, which aryl or heteroaryl group optionally bears one or more substituents independently selected from halogeno, cyano and (1-4C)alkoxy;
**R², R³, R⁴ and R⁵** are each, independently, selected from hydrogen and (1-4C)alkyl, or
R² and R³ together with the carbon atom to which they are attached form a cyclopropyl ring, or
R⁴ and R⁵ together with the carbon atom to which they are attached form a cyclopropyl ring;
**R⁶** is selected from hydrogen and (1-4C)alkyl;
**A** is selected from hydrogen, a group of the formula Z-(CR⁸R⁹)ₚ-and R¹⁰,
   wherein p is 1, 2, 3, or 4,
R⁸ and R⁹ are each, independently, selected from hydrogen and (1-4C)alkyl, or an R⁸ and an R⁹ group attached to the same carbon atom form a cyclopropyl ring,
Z is selected from hydrogen, OR¹¹ and NR¹²R¹³, wherein R¹¹, R¹² and R¹³ are each, independently, selected from hydrogen and (1-4C)alkyl, and
R¹⁰ is selected from (1-4C)alkoxy and NR¹²R¹³, wherein R¹² and R¹³ are as defined above,
   and wherein any CH₂ or CH₃ group within a Z or an RIO group optionally bears on each said CH₂ or CH₃ group one or more substituents independently selected from halogeno, (1-4C)alkyl, hydroxy and (1-4C)alkoxy;
   or a pharmaceutically acceptable salt thereof.

According, to a second aspect of the invention there is provided a quinazoline derivative of the Formula **I** wherein:
**R¹** is selected from hydrogen, hydroxy, (1-4C)alkoxy and (1-4C)alkoxy(1-4C)alkoxy;
**G¹ G², G³, G⁴ and G⁵** are each, independently, selected from hydrogen and halogeno;
**X¹** is selected from SO₂, CO, SO₂N(R⁷) and C(R⁷)₂, wherein each R⁷ is, independently, selected from hydrogen and (1-4C)alkyl;
**Q¹** is aryl or heteroaryl, which aryl or heteroaryl group optionally bears one or more substituents independently selected from halogeno, cyano and (1-4C)alkoxy;
**R², R³, R⁴ and R⁵** are each, independently, selected from hydrogen and (1-4C)alkyl;
**R⁶** is selected from hydrogen and (1-4C)alkyl;
**A** is selected from hydrogen, a group of the formula Z-(CR⁸R⁹)ₚ- and R¹⁰,
   wherein p is 1, 2, 3, or 4,
R⁸ and R⁹ are each, independently, selected from hydrogen and (1-4C)alkyl, or an R⁸ and an R⁹ group attached to the same carbon atom form a cyclopropyl ring,
Z is selected from hydrogen, OR¹¹ and NR¹²R¹³, whereinR¹¹,R¹² and R¹³ are each, independently, selected from hydrogen and (1-4C)alkyl, and
R¹⁰ is selected from (1-4C)alkoxy and NR¹²R¹³, wherein R¹² and R¹³ are as defined above,
   and wherein any CH₂ or CH₃ group within a Z or an R¹⁰ group optionally bears on each said CH₂ or CH₃ group one or more substituents independently selected from halogeno, (1-4C)alkyl, hydroxy and (1-4C)alkoxy;
   or a pharmaceutically acceptable salt thereof.

In this specification the generic term "alkyl" includes both straight-chain and branched-chain alkyl groups such as propyl, isopropyl and tert-butyl. However references to individual alkyl groups such as "propyl" are specific for the straight-chain version only and references to individual branched-chain alkyl groups such as "isopropyl" are specific for the branched-chain version only. An analogous convention applies to other generic terms, for example (1-4C)alkoxy includes methoxy and ethoxy.

It is to be understood that, insofar as certain of the quinazoline derivatives of the Formula **I** defined above may exist in optically active or racemic forms by virtue of one or more asymmetric carbon atoms, the invention includes in its definition any such optically active or racemic form which possesses the above-mentioned activity. In particular, the quinazoline derivative of the Formula **I** may have a chiral centre on the carbon atom attached to the groups R² and R³ and/or on the carbon atom attached to the groups R⁴ and R ⁵ if the groups R² and R³ and/or the groups R⁴ and R⁵ are not identical. The present invention encompasses all such stereoisomers having activity as herein defined, for example the (2R) and (2S) isomers (particularly the (2R) isomers). It is further to be understood that in the names of chiral compounds (*R,S*) denotes any scalemic or racemic mixture while (*R*) and (*S*) denote the enantiomers. In the absence of (*R,S*), (*R*) or (*S*) in the name it is to be understood that the name refers to any scalemic or racemic mixture, wherein a scalemic mixture contains *R* and *S* enantiomers in any relative proportions and a racemic mixture contains *R* and *S* enantiomers in the ratio 50:50. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

Suitable values for the generic radicals referred to above include those set out below.

A suitable value for Q¹ when it is aryl is, for example, phenyl or naphthyl, particularly phenyl.

A suitable value for Q¹ when it is heteroaryl is, for example, an aromatic 5- or 6-membered monocyclic ring with up to 4 ring heteroatoms independently selected from oxygen, nitrogen and sulfur, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl. A particular value for Q¹ when it is heteroaryl is, for example, an aromatic 5- or 6-membered monocyclic ring containing nitrogen and, optionally, 1 or 2 (for example 1) additional ring heteroatoms independently selected from oxygen, nitrogen and sulfur, for example pyrrolyl, oxazolyl, isoxazolyl, imidazolyl, pyrazolyl, thiazolyl, isothiazolyl, oxadiazolyl, thiadiazolyl, triazolyl, pyridyl, pyridazinyl, pyrimidinyl, pyrazinyl or 1,3,5-triazinyl (especially pyridyl).

Suitable values for any of the 'R' groups (R¹ to R¹³), for any of the 'G' groups (G¹ to G⁵) or for various groups within a Q¹, X¹, A or Z group include:-

| | |
|---|---|
| for halogeno | fluoro, chloro, bromo and iodo; |
| for (1-4C)alkyl: | methyl, ethyl, propyl, isopropyl and tert-butyl; |
| for (1-4C)alkoxy: | methoxy, ethoxy, propoxy, isopropoxy and butoxy; and |
| for (1-4C)alkoxy(1-4C)alkoxy | ethoxymethoxy, propoxymethoxy, methoxyethoxy, ethoxyethoxy, methoxypropoxy, |
| | ethoxypropoxy, methoxyisopropoxy and methoxybutoxy. |

When, as defined hereinbefore, in the group of the formula -X¹-Q¹, X¹ is, for example, a SO₂N(R⁷) linking group, it is the SO₂ group of the SO₂N(R⁷) linking group which is attached to the indole group in the Formula **I** and the nitrogen atom which is attached to the Q¹ group.

It is to be understood that certain quinazoline derivatives of the Formula **I** may exist in solvated as well as unsolvated forms such as, for example, hydrated forms. It is to be understood that the invention encompasses all such solvated forms which exhibit an inhibitory effect on an erbB receptor tyrosine kinase, such as anti-proliferative activity.

It is also to be understood that certain quinazoline derivatives of the Formula **I** may exhibit polymorphism, and that the invention encompasses all such forms which exhibit an inhibitory effect on an erbB receptor tyrosine kinase, such as anti-proliferative activity.

It is also to be understood that the invention relates to all tautomeric forms of the quinazoline derivatives of the Formula **I** which exhibit an inhibitory effect on an erbB receptor tyrosine kinase, such as anti-proliferative activity.

A suitable pharmaceutically acceptable salt of a quinazoline derivative of the Formula **I** is, for example, an acid-addition salt of a quinazoline derivative of the Formula **I**, for example an acid-addition salt with an inorganic or organic acid. Suitable inorganic acids, for example, hydrochloric, hydrobromic or sulfuric acid. Suitable organic acids include, for example, trifluoroacetic, citric or maleic acid. Another suitable pharmaceutically acceptable salt of a quinazoline derivative of the Formula **I** is, for example, a salt of a quinazoline derivative of the Formula **I** which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel quinazoline derivatives of the invention include, for example, quinazoline derivatives of the Formula **I**, or pharmaceutically acceptable salts thereof, wherein, unless otherwise stated, each of R¹, R², R³, R⁴, R⁵, R⁶, G¹, G², G³, G⁴, G⁵, Q¹, X¹ and A has any of the meanings defined hereinbefore or in paragraphs (a) to (kkk) hereinafter:
(a) R¹ is selected from hydrogen, hydroxy, methoxy, ethoxy and methoxyethoxy;
(b) R¹ is selected from hydrogen and methoxy;
(c) R¹ is hydrogen;
(d) G¹, G², G³, G⁴ and G⁵ are each, independently, selected from hydrogen, chloro and fluoro;
(e) G¹, G², G³, G⁴ and G⁵ are all hydrogen;
(f) X¹ is C(R⁷)₂, wherein each R⁷ is, independently, selected from hydrogen and (1-4C)alkyl (such as (1-2C)alkyl);
(g) X¹ is CH₂;
(h) Q¹ is selected from phenyl and a 5- or 6-membered monocyclic heteroaryl ring, which ring contains 1, 2 or 3 heteroatoms independently selected from oxygen, nitrogen and sulfur, which phenyl or heteroaryl group optionally bears 1, 2 or 3 substituents (for example 1 or 2) independently selected from halogeno, cyano and (1-4C)alkoxy;
(i) Q¹ is selected from phenyl and a 5- or 6-membered monocyclic heteroaryl ring, which ring contains 1, 2 or 3 heteroatoms independently selected from oxygen, nitrogen and sulfur, which phenyl or heteroaryl group optionally bears 1, 2 or 3 substituents (for example 1 or 2) independently selected from chloro, fluoro, cyano and (1-3C)alkoxy;
(j) Q¹ is phenyl, which phenyl group optionally bears 1, 2 or 3 substituents (for example 1 or 2) as hereinbefore defined in (h) or (i);
(k) Q¹ is phenyl, which phenyl group optionally bears 1 or 2 substituents independently selected from chloro and fluoro;
(l) Q¹ is phenyl, which phenyl group bears 1 or 2 substituents independently selected from chloro and fluoro;
(m) Q¹ is phenyl, which phenyl group bears 1 or 2 (particularly 1) fluoro substituents;
(n) Q¹ is 3-fluorophenyl;
(o) Q¹ is a 5- or 6-membered monocyclic heteroaryl ring, which ring contains 1 nitrogen heteroatom and optionally 1 additional heteroatom selected from oxygen, nitrogen and sulfur, which heteroaryl group optionally bears 1,2 or 3 substituents (for example 1 or 2) as hereinbefore defined in (h) or (i);
(p) Q¹ is selected from phenyl, pyridyl, pyrazinyl, 1,3-thiazolyl, 1H-imidazolyl, 1H-pyrazolyl, 1,3-oxazolyl and isoxazolyl, which optionally bears 1, 2 or 3 substituents (for example 1 or 2) as hereinbefore defined in (h) or (i);
(q) Q¹ is selected from phenyl, pyridyl, pyrazinyl, 1,3-thiazolyl and isoxazolyl, which optionally bears 1, 2 or 3 substituents (for example 1 or 2) as hereinbefore defined in (h) or (i);
(r) Q¹ is selected from 2-, 3- or 4-pyridyl, 2-pyrazinyl, 1,3-thiazol-2-yl, 1,3-thiazol-4-yl, 1,3-thiazol-5-yl, 3-isoxazolyl, 4-isoxazolyl and 5-isoxazolyl, which optionally bears 1, 2 or 3 substituents (for example 1 or 2) as hereinbefore defined in (h) or (i);
(s) Q¹ is selected from phenyl, 2-pyridyl and 1,3-thiazol-4-yl, which optionally bears 1, 2 or 3 substituents (for example 1 or 2) as hereinbefore defined in (h) or (i);
(t) Q¹ is pyridyl (particularly 2-pyridyl or 3-pyridyl, more particularly 2-pyridyl), which optionally bears 1, 2 or 3 substituents (for example 1 or 2) as hereinbefore defined in (h) or (i);
(u) Q¹ is 2-pyridyl, which optionally bears 1 or 2 substituents independently selected from fluoro, chloro and (1-2C)alkoxy;
(v) Q¹ is 2-pyridyl;
(w) Q¹ is 1,3-thiazolyl (particularly 1,3-thiazol-2-yl, 1,3-thiazol-4-yl or 1,3-thiazolyl-5-yl), which optionally bears 1 or 2 substituents (for example 1) as hereinbefore defined in (h) or (i);
(x) Q¹ is 1,3-thiazol-4-yl, which optionally bears 1 or 2 substituents independently selected from fluoro, chloro and (1-2C)alkoxy;
(y) Q¹ is 1,3-thiazol-4-yl;
(z)1,3-thiazol-4-yl, 1,3-thiazol-5-yl, 3-isoxazolyl, 4-isoxazolyl and 5-isoxazolyl, which optionally bears 1, 2 or 3 substituents (for example 1 or 2) as hereinbefore defined in (h) or (i); and
   X¹ is C(R⁷)₂, wherein each R⁷ is, independently, selected from hydrogen and (1-2C)alkyl (particularly each R⁷ is hydrogen);
   (aa) Q¹ is selected from 2-, 3- or 4-pyridyl, 2-pyrazinyl, 1,3-thiazol-2-yl, 1,3-thiazol-4-yl, 1,3-thiazol-5-yl, 3-isoxazolyl, 4-isoxazolyl and 5-isoxazolyl, which optionally bears 1, 2 or 3 substituents (for example 1 or 2) as hereinbefore defined in (h) or (i);
   X¹ is C(R⁷)₂, wherein each R⁷ is, independently, selected from hydrogen and (1-2C)alkyl (particularly each R⁷ is hydrogen); and
   G¹, G², G³, G⁴ and G⁵ are all hydrogen;
(bb) the group -X¹-Q¹ is selected from pyrid-2-ylmethyl, 1,3-thiazol-4-ylmethyl and 3-fluorobenzyl;
(cc) the group -X¹-Q¹ is pyrid-2-ylmethyl;
(dd) R², R³, R⁴ and R⁵ are each, independently, selected from hydrogen and (1-2C)alkyl (such as methyl);
(ee) R², R³, R⁴ and R⁵ are each, independently, selected from hydrogen and (1-2C)alkyl, wherein at least one of R², R³, R⁴ and R⁵ is (1-2C)alkyl (such as methyl);
(ff) R², R³ and R⁴ are all hydrogen and R⁵ is (1-2C)alkyl (such as methyl);
(gg) R², R⁴ and R⁵ are all hydrogen and R³ is (1-2C)alkyl (such as methyl);
(hh) R² and R³ are both hydrogen and R⁴ and R⁵ are both (1-2C)alkyl (such as methyl); (ii) R² and R⁴ are both hydrogen;
(jj) R⁴ and R⁵ are hydrogen, and
   R² and R³ together with the carbon atom to which they are attached form a cyclopropyl ring;
(kk) R² and R³ are hydrogen, and
   R⁴ and R⁵ together with the carbon atom to which they are attached form a cyclopropyl ring;
(ll) R², R³, R⁴ and R⁵ are all hydrogen;
(mm) R⁶ is selected from hydrogen and (1-2C)alkyl;
(nn) R⁶ is methyl;
(oo) R⁶ is hydrogen;
(pp) A is selected from a group of the formula Z-(CR⁸R⁹)ₚ- and R¹⁰,
   wherein p is 1, 2, 3, or 4,
   R⁸ and R⁹ are each, independently, selected from hydrogen and (1-4C)alkyl, or an R⁸ and an R⁹ group attached to the same carbon atom form a cyclopropyl ring,
   Z is selected from hydrogen, OR¹¹ and NR¹²R¹³, wherein R¹¹, R¹² and R¹³ are each, independently, selected from hydrogen and (1-4C)alkyl,
   R¹⁰ is selected from (1-4C)alkoxy and NR¹²R¹³, wherein R¹² and R¹³ are as defined above,
      and wherein any CH₂ or CH₃ group within a Z or an R¹⁰ group optionally bears on each said CH₂ or CH₃ group one or more (for example 1 , 2 or 3) substituents independently selected from halogeno, (1-4C)alkyl, hydroxy and (1-4C)alkoxy;
(qq) A is selected from a group of the formula Z-(CR⁸R⁹)ₚ- and R¹⁰,
   wherein p is 1, 2 or 3 (such as 1 or 2),
   R⁸ and R⁹ are each, independently, selected from hydrogen and (1-2C)alkyl, or an R⁸ and an R⁹ group attached to the same carbon atom form a cyclopropyl ring,
   Z is selected from OR¹¹ and NR¹²R¹³, wherein R¹¹, R¹² and R¹³ are each, independently, selected from hydrogen and (1-2C)alkyl,
   R¹⁰ is selected from (1-2C)alkoxy and NR¹²R¹³, wherein R¹² and R¹³ are as defined above,
      and wherein any CH₂ or CH₃ group within a Z or an R¹⁰ group optionally bears on each said CH₂ or CH₃ group one or more (for example 1, 2 or 3) substituents independently selected from halogeno, (1-2C)alkyl, hydroxy and (1-2C)alkoxy;
(rr) A is a group of the formula Z-(CR⁸R⁹)ₚ-,
   wherein p is 1 or 2,
   R⁸ and R⁹ are each, independently, selected from hydrogen and (1-4C)alkyl, or an R⁸ and an R⁹ group attached to the same carbon atom form a cyclopropyl ring,
   Z is selected from hydrogen, OR¹¹ and NR¹²R¹³, wherein R¹¹, R¹² and R¹³ are each, independently, selected from hydrogen and (1-4C)alkyl,
      and wherein any CH₂ or CH₃ group within a Z group optionally bears on each said CH₂ or CH₃ group one or more (for example 1, 2 or 3) substituents independently selected from halogeno, (1-2C)alkyl, hydroxy and (1-2C)alkoxy;
(ss) A is a group of the formula Z-CCR⁸R⁹)ₚ-,
   wherein p is 1 or 2,
   R⁸ and R⁹ are each, independently, selected from hydrogen and (1-4C)alkyl,
   Z is selected from hydrogen, OR¹¹ and NR¹²R¹³, wherein R¹¹, R¹² and R¹³ are each, independently, selected from hydrogen and (1-4C)alkyl,
      and wherein any CH₂ or CH₃ group within a Z group optionally bears on each said CH₂ or CH₃ group one or more (for example 1, 2 or 3) substituents independently selected from halogeno, (1-2C)alkyl and hydroxy;
(tt) A is a group of the formula Z-(CR⁸R⁹)ₚ-,
   wherein p is 1 or 2,
   R⁸ and R⁹ are each, independently, selected from hydrogen and (1-4C)alkyl, or an R⁸ and an R⁹ group attached to the same carbon atom form a cyclopropyl ring,
   Z is selected from hydrogen and OR¹¹, wherein R¹¹ is selected from hydrogen and (1-4C)alkyl,
      and wherein any CH₂ or CH₃ group within a Z group optionally bears on each said CH₂ or CH₃ group one or more (for example 1, 2 or 3) substituents independently selected from halogeno, (1-2C)alkyl, hydroxy and (1-2C)alkoxy;
(uu) A is a group of the formula Z-(CR⁸R⁹)ₚ-,
   wherein p is 1 or 2,
   R⁸ and R⁹ are each, independently, selected from hydrogen and (1-4C)alkyl, or an R⁸ and an R⁹ group attached to the same carbon atom form a cyclopropyl ring, and
   Z is hydroxy;
(vv) A is a group of the formula Z-(CR⁸R⁹)ₚ-,
   wherein p is 1 or 2,
   R⁸ and R⁹ are each, independently, selected from hydrogen and (1-2C)alkyl, and
   Z is hydroxy;
(ww) A is a group of the formula Z-(CR⁸R⁹)ₚ-,
   wherein p is 1 or 2,
   R⁸ and R⁹ are each, independently, selected from hydrogen and (1-4C)alkyl, or an R⁸ and an R⁹ group attached to the same carbon atom form a cyclopropyl ring,
   Z is NR¹²R¹³, wherein R¹² and R¹³ are each, independently, selected from hydrogen and (1-4C)alkyl,
      and wherein any CH₂ or CH₃ group within a Z group optionally bears on each said CH₂ or CH₃ group one or more (for example 1, 2 or 3) substituents independently selected from halogeno, (1-2C)alkyl, hydroxy and (1-2C)alkoxy;
(xx) A is a group of the formula Z-(CR⁸R⁹)ₚ-,
   wherein p is 1 or 2,
   R⁸ and R⁹ are each, independently, selected from hydrogen and (1-4C)alkyl, or an R⁸ and an R⁹ group attached to the same carbon atom form a cyclopropyl ring,
      provided (i) that at least one of the R⁸ or R⁹ groups is (1-4C)alkyl, or (ii) that an R⁸ and an R⁹ group attached to the same carbon atom form a cyclopropyl ring,
   Z is selected from hydrogen, OR¹¹ and NR¹²R¹³, wherein R¹¹, R¹² and R¹³ are each, independently, selected from hydrogen and (1-4C)alkyl,
      and wherein any CH₂ or CH₃ group within a Z group optionally bears on each said CH₂ or CH₃ group one or more (for example 1, 2 or 3) substituents independently selected from halogeno, (1-4C)alkyl, hydroxy and (1-4C)alkoxy;
(yy) A is R¹⁰, wherein R¹⁰ is selected from (1-4C)alkoxy and NR¹²R¹³, wherein R¹² and R¹³ are each, independently, selected from hydrogen and (1-4C)alkyl,
   and wherein any CH₂ or CH₃ group within an R¹⁰ group optionally bears on each said CH₂ or CH₃ group one or more (for example 1, 2 or 3) substituents independently selected from halogeno, (1-4C)alkyl, hydroxy and (1-4C)alkoxy;
(zz) A is R¹⁰, wherein R¹⁰ is (1-4C)alkoxy (particularly (1-2C)alkoxy, such as methoxy),
   and wherein any CH₂ or CH₃ group within an R¹⁰ group optionally bears on each said CH₂ or CH₃ group one or more (for example 1, 2 or 3) substituents independently selected from halogeno, (1-2C)alkyl, hydroxy and (1-2C)alkoxy;
(aaa) A is R¹⁰, wherein R¹⁰ is NR¹²R¹³, wherein R¹² and R¹³ are each, independently, selected from hydrogen and (1-4C)alkyl,
   and wherein any CH₂ or CH₃ group within an R¹⁰ group optionally bears on each said CH₂ or CH₃ group one or more (for example 1, 2 or 3) substituents independently selected from halogeno, (1-2C)alkyl, hydroxy and (1-2C)alkoxy;
(bbb) A is selected from methyl, ethyl, propyl, isopropyl, hydroxymethyl, 2-hydroxyethyl, 1-hydroxyethyl, 3-hydroxypropyl, 1-hydroxypropyl, 2-hydroxypropyl, 2-hydroxyprop-2-yl,1,3-dihydroxypropyl,2-(hydroxymethyl)prop-2-yl,2-hydroxy-2-methylpropyl, methoxymethyl, 2-methoxyethyl, 1-methoxyethyl, 3-methoxypropyl, 1-methoxypropyl, 2-methoxypropyl, 2-methoxyprop-2-yl, 2-(methoxymethyl)prop-2-yl, 2-methoxy-2-methylpropyl, ethoxymethyl, 2-ethoxyethyl, 1-ethoxyethyl, 1-hydroxy-3-bromopropyl, aminomethyl, 2-aminoethyl, 1-aminoethyl, 3-aminopropyl, 1-aminopropyl, 2-aminopropyl, 2-aminoprop-2-yl, 2-(aminomethyl)prop-2-yl, 2-amino-2-methylpropyl, N-methylaminomethyl, 2-(N-methylamino)ethyl, 1-(N-methylamino)ethyl, 3-(N-methylamino)propyl, 1-(N-methylamino)propyl, 2-(N-methylamino)propyl, 2-(N-methylamino)prop-2-yl, 2-(N-methylaminomethyl)prop-2-yl, 2-(N-methylamino)-2-methylpropyl, N,N-dimethylaminomethyl, 2-(N,N-dimethylamino)ethyl, 1-(N,N-dimethylamino)ethyl, 3-(N,N-dimethylamino)propyl, 1-(N,N-dimethylamino)propyl, 2-(N,N-dimethylamino)propyl, 2-(N,N-dimethylamino)prop-2-yl, 2-(N,N-dimethylaminomethyl)prop-2-yl, 2-(N,N-dimethylamino)-2-methylpropyl, methylamino, dimethylamino, ethylamino, diethylamino, (2-chloroethyl)amino, methoxy, ethoxy, propoxy, butoxy, cyclopropyl and 1-hydroxycyclopropyl;
(ccc) A is selected from methyl, ethyl, propyl, isopropyl, hydroxymethyl, 2-hydroxyethyl, 1-hydroxyethyl, 3-hydroxypropyl, 1-hydroxypropyl, 2-hydroxypropyl, 2-hydroxyprop-2-yl, 2-(hydroxymethyl)prop-2-yl, 2-hydroxy-2-methylpropyl, methoxymethyl, 2-methoxyethyl, 1-methoxyethyl, 3-methoxypropyl, 1-methoxypropyl, 2-methoxypropyl, 2-methoxyprop-2-yl, 2-(methoxymethyl)prop-2-yl, 2-methoxy-2-methylpropyl, ethoxymethyl, 2-ethoxyethyl, 1-ethoxyethyl, aminomethyl, 2-aminoethyl, 1-aminoethyl, 3-aminopropyl, 1-aminopropyl, 2-aminopropyl, 2-aminoprop-2-yl, 2-(aminomethyl)prop-2-yl, 2-amino-2-methylpropyl, N-methylaminomethyl, 2-(N-methylamino)ethyl, 1-(N-methylamino)ethyl, 3-(N-methylamino)propyl, 1-(N-methylamino)propyl, 2-(N-methylamino)propyl, 2-(N-methylamino)prop-2-yl, 2-(N-methylaminomethyl)prop-2-yl, 2-(N-methylamino)-2-methylpropyl, N,N-dimethylaminomethyl, 2-(N,N-dimethylamino)ethyl, 1-(N,N-dimethylamino)ethyl, 3-(N,N-dimethylamino)propyl, 1-(N,N-dimethylamino)propyl, 2-(N,N-dimethylamino)propyl, 2-(N,N-dimethylamino)prop-2-yl, 2-(N,N-dimethylaminomethyl)prop-2-yl, 2-(N,N-dimethylamino)-2-methylpropyl, cyclopropyl and 1-hydroxycyclopropyl;
(ddd) A is selected from methyl, ethyl, propyl, isopropyl, hydroxymethyl, 2-hydroxyethyl,1-hydroxyethyl,3-hydroxypropyl,1-hydroxypropyl,2-hydroxypropyl, 2-hydroxyprop-2-yl, 1,3-dihydroxypropyl, 2-(hydroxymethyl)prop-2-yl, 2-hydroxy-2-methylpropyl, methoxymethyl, 2-methoxyethyl, 1-methoxyethyl, 3-methoxypropyl, 1-methoxypropyl, 2-methoxypropyl, 2-methoxyprop-2-yl, 1-hydroxy-3-bromopropyl, aminomethyl, 2-aminoethyl, 1-aminoethyl, 3-aminopropyl, 1-aminopropyl, 2-aminopropyl, 2-aminoprop-2-yl, 2-(aminomethyl)prop-2-yl, 2-amino-2-methylpropyl, N-methylaminomethyl, 2-(N-methylamino)ethyl, 1-(N-methylamino)ethyl, N,N-dimethylaminomethyl, 2-(N,N-dimethylamino)ethyl, 1-(N,N-dimethylamino)ethyl, methylamino, dimethylamino, ethylamino, diethylamino, (2-chloroethyl)amino, methoxy, ethoxy, cyclopropyl and 1-hydroxycyclopropyl;
(eee) A is selected from methyl, hydroxymethyl, 2-hydroxyethyl, 1-hydroxyethyl, 3-hydroxypropyl, 1,3-dihydroxypropyl, 2-(hydroxymethyl)prop-2-yl, methoxymethyl, 1-methoxyethyl, 1-hydroxy-3-bromopropyl, aminomethyl, N-methylaminomethyl, methylamino, (2-chloroethyl)amino, methoxy and 1-hydroxycyclopropyl;
(fff) A is selected from hydroxymethyl, 2-hydroxyethyl, 1-hydroxyethyl, 3-hydroxypropyl, 1-hydroxypropyl, 2-hydroxypropyl, 2-hydroxyprop-2-yl, 2-(hydroxymethyl)prop-2-yl and 2-hydroxy-2-methylpropyl;
(ggg) A is selected from methyl, hydroxymethyl, 2-hydroxyethyl, 1-hydroxyethyl and 2-hydroxyprop-2-yl;
(hhh) A is selected from methyl and hydroxymethyl;
(iii) A is hydroxymethyl;
(jjj) A is selected from aminomethyl, 2-aminoethyl, 1-aminoethyl, 3-aminopropyl, 1-aminopropyl, 2-aminopropyl, 2-aminoprop-2-yl, 2-(aminomethyl)prop-2-yl, 2-amino-2-methylpropyl, N-methylaminomethyl, 2-(N-methylamino)ethyl, 1-(N-methylamino)ethyl, 3-(N-methylamino)propyl, 1-(N-methylamino)propyl, 2-(N-methylamino)propyl, 2-(N-methylamino)prop-2-yl, 2-(N-methylaminomethyl)prop-2-yl, 2-(N-methylamino)-2-methylpropyl, N,N-dimethylaminomethyl, 2-(N,N-dimethylamino)ethyl, 1-(N,N-dimethylamino)ethyl, 3-(N,N-dimethylamino)propyl, 1-(N,N-dimethylamino)propyl, 2-(N,N-dimethylamino)propyl, 2-(N,N-dimethylamino)prop-2-yl, 2-(N,N-dimethylaminomethyl)prop-2-yl and 2-(N,N-dimethylamino)-2-methylpropyl; and
(kkk) A is selected from aminomethyl, 2-aminoethyl, N-methylaminomethyl, 2-(N-methylamino)ethyl, N,N-dimethylaminomethyl and 2-(N,N-dimethylamino)ethyl.
   An embodiment of the present invention is a quinazoline derivative of the Formula **I** wherein:
   **R¹** is selected from hydrogen and (1-2C)alkoxy (for example R¹ is hydrogen or methoxy, particularly hydrogen);
   **X¹** is CH₂;
   **Q¹** is aryl or heteroaryl, which aryl or heteroaryl group optionally bears one or more substituents (for example 1 or 2) independently selected from chloro, fluoro, cyano and (1-2C)alkoxy;
      and wherein G¹, G², G³, G⁴, G⁵, R², R³, R⁴, R⁵, R⁶ and A have any of the values defined hereinbefore;
      or a pharmaceutically acceptable salt thereof.

In this embodiment a particular value for Q¹ is phenyl or a 5- or 6-membered heteroaryl ring containing 1 nitrogen heteroatom and optionally 1 additional heteroatom independently selected from oxygen, nitrogen and sulfur, which phenyl or heteroaryl group optionally bears 1, 2 or 3 substituents as hereinbefore defined. A more particular value for Q¹ is pyridyl, which optionally bears 1, 2 or 3 substituents as hereinbefore defined.

Another embodiment of the present invention is a quinazoline derivative of the Formula **I** wherein:
**R¹** is selected from hydrogen and (1-2C)alkoxy (for example R¹ is hydrogen or methoxy, particularly hydrogen);
**X¹** is CH₂;
**Q¹** is heteroaryl, which heteroaryl group optionally bears one or more substituents (for example 1 or 2) independently selected from chloro, fluoro, cyano and (1-2C)alkoxy;
   and wherein G¹, G², G³, G⁴, G⁵, R², R³, R⁴, R⁵, R⁶ and A have any of the values defined hereinbefore;
   or a pharmaceutically acceptable salt thereof.

In this embodiment a particular value for Q¹ is a 5- or 6-membered heteroaryl ring containing 1 nitrogen heteroatom and optionally 1 additional heteroatom independently selected from oxygen, nitrogen and sulfur, which heteroaryl group optionally bears 1, 2 or 3 substituents as hereinbefore defined. A more particular value for Q¹ is pyridyl, which optionally bears 1, 2 or 3 substituents as hereinbefore defined.

Another embodiment of the present invention is a quinazoline derivative of the Formula **I** wherein:
**R¹** is selected from hydrogen and (1-2C)alkoxy (for example R¹ is hydrogen or methoxy, particularly hydrogen);
**X¹** is CH₂;
**Q¹** is phenyl or a 5- or 6-membered heteroaryl ring containing 1 nitrogen heteroatom and optionally 1 additional heteroatom independently selected from oxygen, nitrogen and sulfur;
**A** is a group of the formula Z-(CR⁸R⁹)ₚ-, wherein p is 1 or 2, R⁸ and R⁹ are each, independently, selected from hydrogen and (1-2C)alkyl and Z is selected from OR¹¹ and NR¹²R¹³, wherein R¹¹, R¹² and R¹³ are each, independently, selected from hydrogen and (1-2C)alkyl;
   and wherein G¹, G², G³, G⁴, G⁵, R², R³, R⁴, R⁵ and R⁶ have any of the values defined hereinbefore;
   or a pharmaceutically acceptable salt thereof.

In this embodiment a particular value for Q¹ is phenyl, pyridyl, pyrazinyl, 1,3-thiazolyl or isoxazolyl, more particularly Q¹ is selected from 2-pyridyl, 3-pyridyl, 2-pyrazinyl, 1,3-thiazol-2-yl, 1,3-thiazol-4-yl, 1,3-thiazol-5-yl and 3-isoxazolyl (especially 2-pyridyl), wherein Q¹ optionally bears 1, 2 or 3 substituents as hereinbefore defined.

An embodiment of the quinazoline derivatives of the Formula I is a quinazoline derivative of the Formula **Ia:** 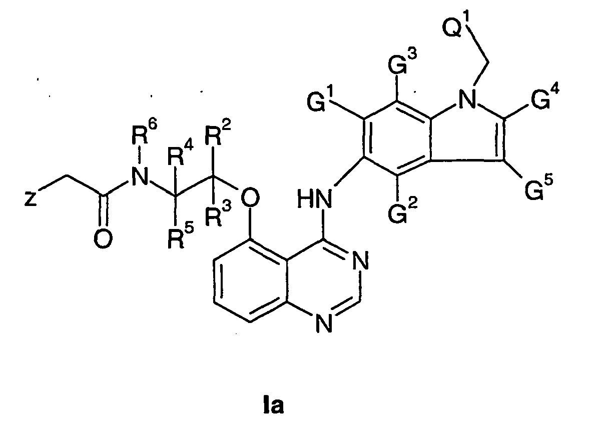 wherein:
**G¹,G²,G³,G⁴ and G⁵** are each, independently, selected from hydrogen and halogeno;
**Q¹** is aryl or heteroaryl, which aryl or heteroaryl group optionally bears one or more substituents independently selected from halogeno, cyano and (1-4C)alkoxy,
**R², R³, R⁴ and R⁵** are each, independently, selected from hydrogen and (1-4C)alkyl, or
R² and R³ together with the carbon atom to which they are attached form a cyclopropyl ring, or
R⁴ and R⁵ together with the carbon atom to which they are attached form a cyclopropyl ring;
**R⁶** is selected from hydrogen and (1-4C)alkyl;
**Z** is selected from hydrogen, OR¹¹ and NR¹²R¹³, wherein R¹¹, R¹² and R¹³ are each, independently, selected from hydrogen and (1-4C)alkyl, and
   and wherein any CH₂ or CH₃ group within a Z group optionally bears on each said CH₂ or CH₃ group one or more substituents independently selected from halogeno, (1-4C)alkyl, hydroxy and (1-4C)alkoxy;
   or a pharmaceutically acceptable salt thereof.

A particular value for Z in the quinazoline derivatives of the Formula **Ia** is hydroxy.

A further embodiment of the quinazoline derivatives of the Formula **I** is a quinazoline derivative of the Formula **Ib:** 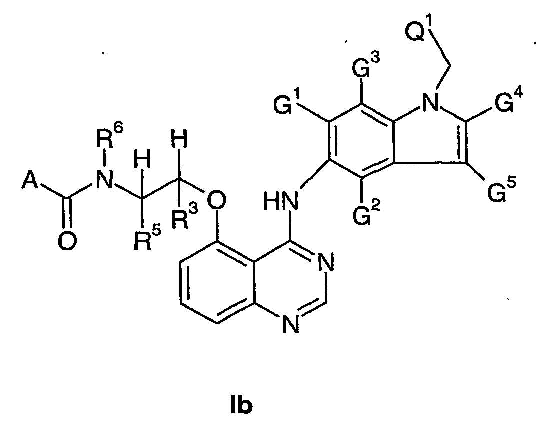 wherein:
**G¹ G², G³, G⁴ and G⁵** are each, independently, selected from hydrogen and halogeno;
**Q¹** is aryl or heteroaryl, which aryl or heteroaryl group optionally bears one or more substituents independently selected from halogeno, cyano and (1-4C)alkoxy;
**R³ and R⁵** are each, independently, selected from hydrogen and (1-4C)alkyl;
**R⁶ is** selected from hydrogen and (1-4C)alkyl;
**A** is selected from hydrogen, a group of the formula Z-(CR⁸R⁹)ₚ- and R¹⁰,
   wherein p is 1, 2, 3, or 4,
R⁸ and R⁹ are each, independently, selected from hydrogen and (1-4C)alkyl, or an R⁸ and an R⁹ group attached to the same carbon atom form a cyclopropyl ring,
Z is selected from hydrogen, OR¹¹ and NR¹²R¹³, wherein R¹¹, R¹² and R¹³ are each, independently, selected from hydrogen and (1-4C)alkyl, and
R¹⁰ is selected from (1-4C)alkoxy and NR¹²R¹³, wherein R¹² and R¹³ are as defined above,
   and wherein any CH₂ or CH₃ group within a Z or an R¹⁰ group optionally bears on each said CH₂ or CH₃ group one or more substituents independently selected from halogeno, (1-4C)alkyl, hydroxy and (1-4C)alkoxy;
   or a pharmaceutically acceptable salt thereof.

A particular value for Z in the quinazoline derivatives of the Formula **Ib** is hydroxy.

In particular, in the quinazoline derivatives of the Formula **Ib,** one of the groups R³ and R⁵ is (1-4C)alkyl (for example (1-2C)alkyl, such as methyl)

For the avoidance of any doubt, in the quinazoline derivatives of the Formulae **Ia** and **Ib,** the group that corresponds to R¹ in the quinazoline derivatives of the Formula **I** is hydrogen.

Particular quinazoline derivatives of the invention are, for example, one or more quinazoline derivatives of the Formula **I** selected from:
2-hydroxy-N-methyl-N-{(2R)-2-[(4-{[1-(pyridin-2-ylmethyl)-1H-indol-5-yl]amino}quinazolin-5-yl)oxy]propyl} acetamide; and
2-hydroxy-N-methyl-N-{(1R)-1-methyl-2-[(4-{[1-(pyridin-2-ylmethyl)-1H-indol-5-yl]amino}quinazolin-5-yl)oxy]ethyl}acetamide;
   or a pharmaceutically acceptable salt thereof.

A quinazoline derivative of the Formula **I,** or a pharmaceutically acceptable salt thereof, may be prepared by any process known to be applicable to the preparation of chemically-related compounds. Suitable processes include, for example, those illustrated in WO 96/15118, WO 01/94341, WO 03/040108 and WO 03/040109. Such processes, when used to prepare a quinazoline derivative of the Formula **I** are provided as a further feature of the invention and are illustrated by the following representative process variants in which, unless otherwise stated, R¹, R², R³, R⁴, R⁵, R⁶, X¹, Q¹, G¹, G², G³, G⁴, G⁵ and A have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.
Process (a) The coupling, conveniently in the presence of a suitable base, of a quinazoline of the Formula **II:** 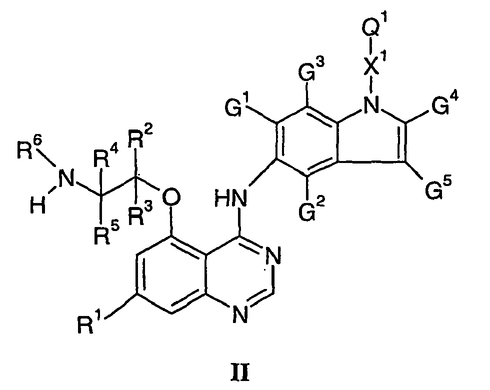 wherein R¹, R², R³, R⁴, R⁵, R⁶, X¹, Q¹, G¹, G², G³, G⁴ and G⁵ have any of the meanings defined hereinbefore except that any functional group is protected if necessary, with a carboxylic acid of the Formula **III,** or a reactive derivative thereof:

   A-COOH **III**

   wherein A has any of the meanings defined hereinbefore except that any functional group is protected if necessary;
   or
Process (b) For the preparation of those quinazoline derivatives of the Formula **I** wherein A is a group of the formula Z-(CR⁸R⁹)ₚ- and Z is NR¹²R¹³, the coupling of a quinazoline of the Formula **IV:** 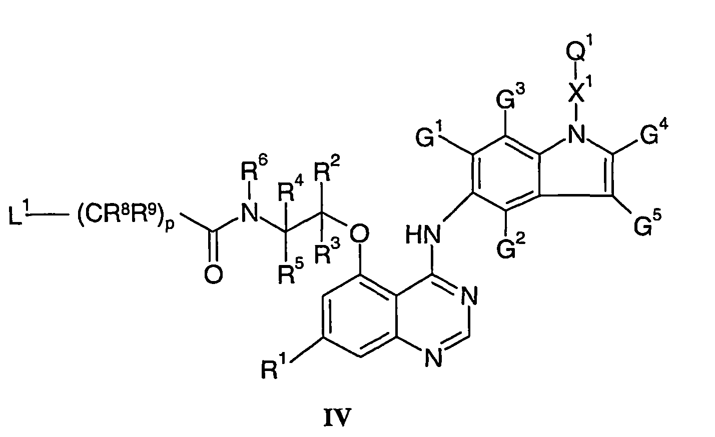 wherein L¹ is a suitable displaceable group and p, R¹, R², R³, R⁴, R⁵, R⁶, R⁸, R⁹, X¹, Q¹, G¹, G², G³, G⁴ and G⁵ have any of the meanings defined hereinbefore except that any functional group is protected if necessary, with an amine of the Formula **V**:

   R¹²R¹³N-H **V**

   wherein R¹² and R¹³ have any of the meanings defined hereinbefore except that any functional group is protected if necessary; or
Process (c) The coupling, conveniently in the presence of a suitable base, of a quinazoline of the Formula **VI:** 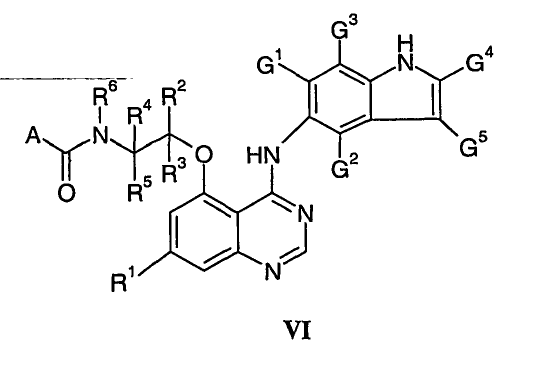 wherein R¹, R², R³, R⁴, R⁵, R⁶, A, G¹, G², G³, G⁴ and G⁵ have any of the meanings defined hereinbefore except that any functional group is protected if necessary, with a compound of the Formula **VII:**

   Q¹-X¹-L² **VII**

   wherein L² is a suitable displaceable group and Q¹ and X¹ have any of the meanings defined hereinbefore except that any functional group is protected if necessary;
Process (d) The coupling, conveniently in the presence of a suitable base, of a quinazoline of the Formula **VIII:** 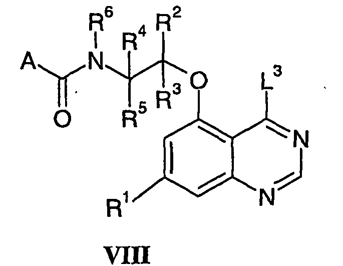 wherein L³ is a suitable displaceable group and R¹, R², R³, R⁴, R⁵, R⁶ and A have any of the meanings defined hereinbefore except that any functional group is protected if necessary, with a compound of the Formula **IX:** 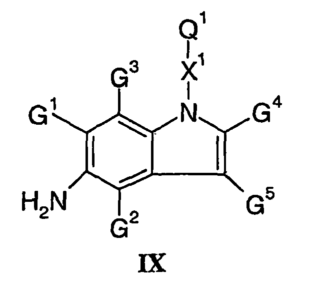 wherein G¹, G², G³, G⁴, G⁵, Q¹ and X¹ have any of the meanings defined hereinbefore except that any functional group is protected if necessary; and thereafter, if necessary:
   (i) converting a quinazoline derivative of the Formula **I** into another quinazoline derivative of the Formula **I;**
   (ii) removing any protecting group that is present (by conventional means);
   (iii) forming a pharmaceutically acceptable salt.

Specific conditions for the above reactions are as follows:

### Process (a)

### Reaction Conditions for Process (a)

As the skilled person would appreciate, the coupling reaction may, if necessary, conveniently be carried out in the presence of a suitable coupling agent, such as a carbodiimide, or a suitable peptide coupling agent, for example O-(7-azabenzotriazol-1-yl)-N,N,N',N'-tetramethyluronium hexafluoro-phosphate (HATU) or a carbodiimide such as dicyclohexylcarbodiimide, optionally in the presence of a catalyst such as dimethylaminopyridine or 4-pyrrolidinopyridine .

The coupling reaction is conveniently carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, such as sodium carbonate, potassium carbonate, cesium carbonate or calcium carbonate.

The reaction is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C, conveniently at or near ambient temperature.

By the term "reactive derivative" of a carboxylic acid of the Formula **III** is meant a carboxylic acid derivative that will react with a quinazoline of the Formula **II** to give the corresponding amide. A suitable reactive derivative of a carboxylic acid of the Formula **III** is, for example, an acyl halide, for example an acyl chloride formed by the reaction of the acid and an inorganic acid chloride, for example thionyl chloride; a mixed anhydride, for example an anhydride formed by the reaction of the acid and a chloroformate such as isobutyl chloroformate; an active ester, for example an ester formed by the reaction of the acid and a phenol such as pentafluorophenol, an ester such as pentafluorophenyl trifluoroacetate or an alcohol such as methanol, ethanol, isopropanol, butanol or N-hydroxybenzotriazole; an acyl azide, for example an azide formed by the reaction of the acid and azide such as diphenylphosphoryl azide; or an acyl cyanide, for example a cyanide formed by the reaction of an acid and a cyanide such as diethylphosphoryl cyanide. The reaction of such reactive derivatives of carboxylic acid with amines (such as a compound of the Formula **II)** is well known in the art, for example they may be reacted in the presence of a base, such as those described above, and in a suitable solvent, such as those described above. The reaction may conveniently be performed at a temperature as described above.

### Preparation of Starting Materials for Process (a)

A quinazoline of the Formula **II** may be obtained by conventional procedures. For example, a quinazoline of the Formula **II** may be obtained by the reaction, conveniently in the presence of a suitable base, of a quinazoline of the Formula **IIa:** 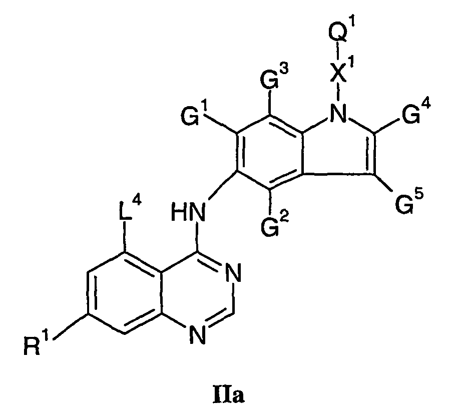 wherein L⁴ is a suitable displaceable group and R¹, X¹, Q¹, G¹, G², G³, G⁴ and G⁵ have any of the meanings defined hereinbefore except that any functional group is protected if necessary, with an alcohol of the Formula **IIb:** 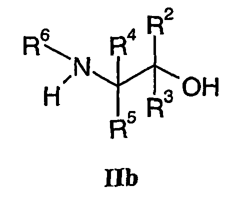 wherein R², R³, R⁴, R⁵ and R⁶ have any of the meanings defined hereinbefore except that any functional group is protected if necessary; and thereafter, if necessary removing any protecting group that is present by conventional means. For example, instead of using the alcohol of the Formula **IIb,** an alcohol of the Formula **IIb'** (including a protecting group, Pg) could be used: 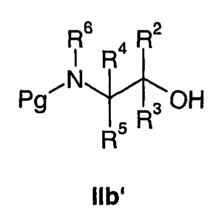 wherein R², R³, R⁴, R⁵ and R⁶ have any of the meanings defined hereinbefore except that any functional group is protected if necessary, following removal of the protecting group (Pg), by an appropriate method known to a person skilled in the art.

A suitable displaceable group L⁴ in a quinazoline of the Formula **IIa** is, for example, halogeno or a sulfonyloxy group, for example fluoro, chloro, methylsulfonyloxy or toluene-4-sulfonyloxy group. A particular displaceable group L⁴ is fluoro or chloro, more particularly fluoro.

A suitable base for the reaction of a quinazoline of the Formula **IIa** and an alcohol of the Formula **IIb** or **IIb'** includes, for example a strong non-nucleophilic base such as an alkali metal hydride, for example sodium hydride, or an alkali metal amide, for example lithium di-isopropylamide (LDA).

The reaction of a quinazoline of the Formula **IIa** and an alcohol of the Formula **IIb** or **IIb'** is conveniently carried out in the presence of a suitable inert solvent or diluent, for example an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range of, for example, 10 to 250°C, preferably in the range 40 to 150°C. Conveniently, this reaction may also be performed by heating the reactants in a sealed vessel using a suitable heating apparatus such as a microwave heater.

Conveniently, the reaction of a quinazoline of the Formula **IIa** and an alcohol of the Formula **IIb** or **IIb'** may be performed in the presence of a suitable catalyst, for example a crown ether such as 15-crown-5.

Alcohols of the Formula **IIb** or **IIb'** are commercially available compounds or they are known in the literature, or they can be can be prepared by standard processes known in the art. For example, alcohols of the Formula **IIb** or **IIb'** wherein R² and R³ are both hydrogen may be prepared by the reduction of the corresponding acid or ester thereof as illustrated in *Reaction Scheme 1:* 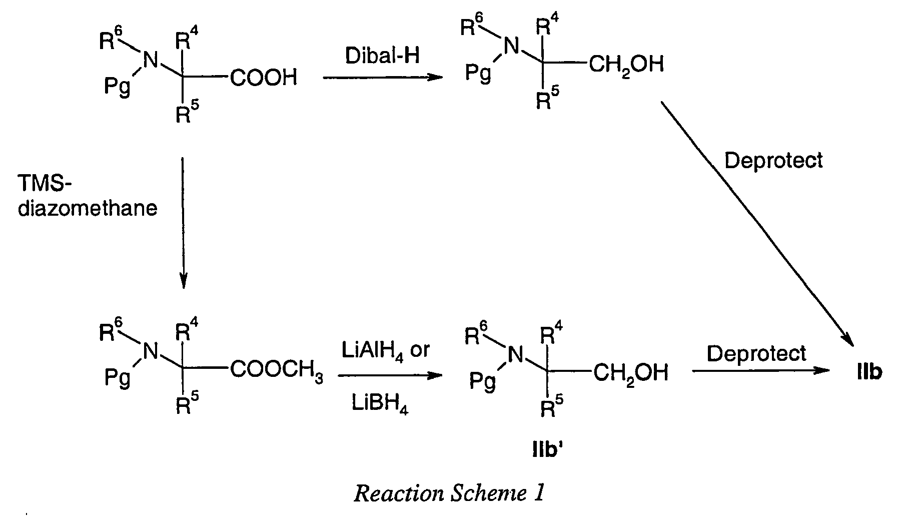 wherein R⁴, R⁵ and R⁶ are as hereinbefore defined, Pg represents a suitable protecting group (such as allyl or tert-butoxy carbonyl), TMS represents trimethylsilane and Dibal-H represents diisobutylaluminium hydride.

In *Reaction Scheme 1*, the reaction with TMS-diazomethane may conveniently be carried out in the presence of methanol, optionally in the presence of a suitable inert solvent or diluent, and at a temperature of about 25°C.

In *Reaction Scheme 1*, the reaction with DiBal-H, LiAlH₄ or LiBH₄ may conveniently be carried out in the presence of a suitable inert solvent or diluent, such as diethyl ether or tetrahydrofuran, and at a temperature in the range, for example, -78 to 60°C.

Alcohols of the Formula **IIb** or **IIb'** alternatively may be prepared as illustrated in *Reaction Scheme 2:* 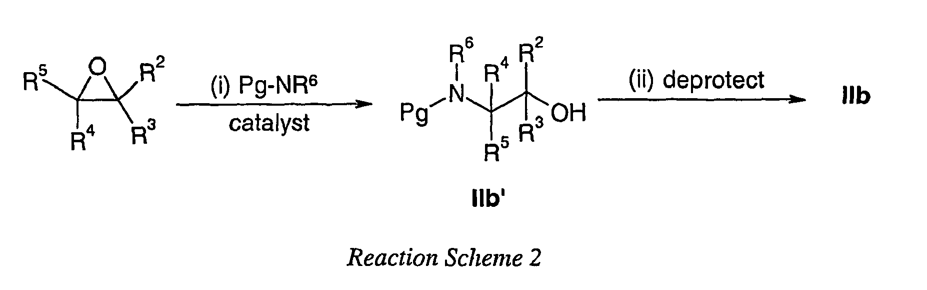 wherein Pg is a suitable amine protecting group (such as allyl), and R², R³, R⁴, R⁵ and R⁶ are as hereinbefore defined.

The coupling and ring opening reaction of step (i) of *Reaction Scheme 2* is conveniently carried out in the presence of a suitable metal catalyst, such as ytterbium(III) trifluoromethanesulfonate. The reaction is suitably carried out in the presence of an inert solvent or diluent such as dioxane. The reaction is preferably carried out at an elevated temperature, for example from 50 to about 150°C.

In step (ii) of *Reaction Scheme 2*, the protecting group Pg may be removed using conventional methods, for example when Pg is an allyl group it may be removed by metal catalysed cleavage. A suitable catalyst for the metal catalysed cleavage is, for example, chlorotris(triphenylphosphine)rhodium (I).

As previously discussed, in some embodiments, the alcohol of the Formula **IIb'** in *Reaction Scheme 2* may be used directly in Process (a). In this embodiment, the amine protecting group (Pg) may be removed at a convenient stage in the process prior to coupling the acid (or reactive derivative thereof) of the Formula **III.**

A quinazoline of the Formula **IIa** may be obtained by conventional procedures. For example, a quinazoline of the Formula **II**c**:** 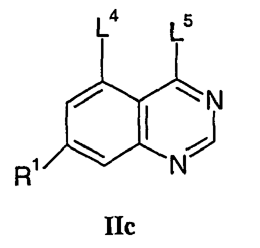 wherein R¹ is as hereinbefore defined and L⁴ and L⁵ are displaceable groups, and L⁵ is more labile than L⁴, may be reacted with a compound of the Formula **IId:** 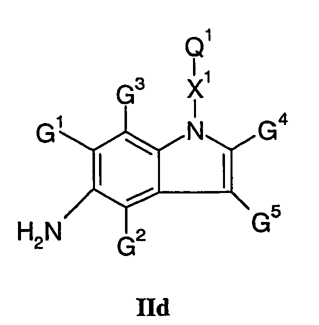 wherein X¹, Q¹ G¹, G², G³, G⁴ and G⁵ have any of the meanings defined hereinbefore except that any functional group is protected if necessary, whereafter any protecting group that is present is removed by conventional means.

A suitable displaceable group L⁴ is as hereinbefore defined, particularly fluoro. A suitable displaceable group L⁵ is, for example, a halogeno (particularly chloro), alkoxy, aryloxy, mercapto, alkylthio, arylthio, alkylsulfinyl, arylsulfinyl, alkylsulfonyl, arylsulfonyl, alkylsulfonyloxy or arylsulfonyloxy group, for example a chloro, bromo, methoxy, phenoxy, pentafluorophenoxy, methylthio, methanesulfonyl, methanesulfonyloxy or toluene-4-sulfonyloxy group.

The reaction of a quinazoline of the Formula **IIc** with a compound of the Formula **IId** may conveniently be carried out in the presence of a catalytic amount of an acid. Suitable acids include, for example hydrogen chloride gas (conveniently dissolved in diethyl ether or dioxane) or hydrochloric acid.

Alternatively, the reaction of a quinazoline of the Formula **IIc** with a compound of the Formula **IId** may be carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, such as sodium carbonate, potassium carbonate, cesium carbonate or calcium carbonate, or, for example, an alkali metal hydride, such as sodium hydride.

Alternatively a quinazoline of the Formula **IIc,** wherein L⁵ is halogeno (for example chloro) may be reacted with a compound of the Formula **IId** in the absence of an acid or a base. In this reaction displacement of the halogeno leaving group L⁵ results in the formation of the acid HL⁵ in-situ and the autocatalysis of the reaction.

The above reactions are conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The above reactions are conveniently carried out at a temperature in the range, for example, 0 to 250°C, conveniently in the range 40 to 80°C or, preferably, at or near the reflux temperature of the solvent when used.

Alternatively, a quinazoline of the Formula **IIa** may be obtained as illustrated in *Reaction Scheme 3:* 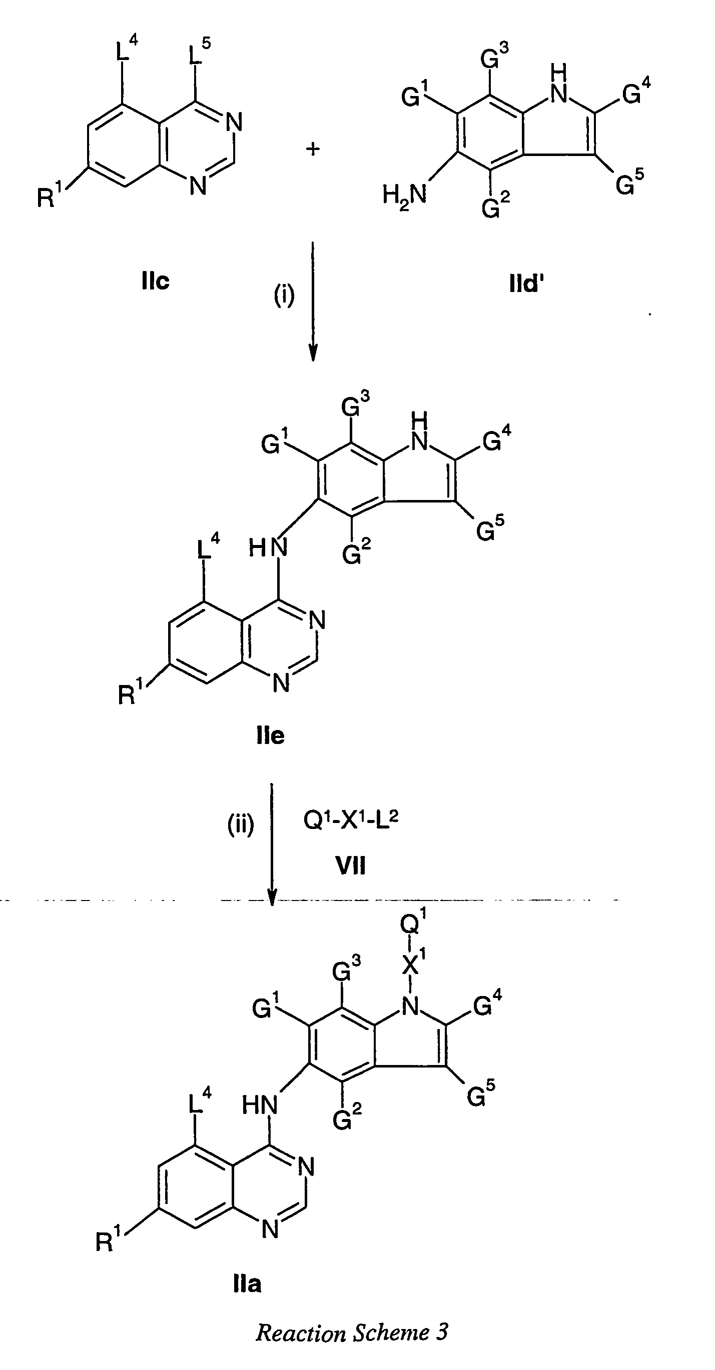 wherein L², L⁴ and L⁵ are suitable displaceable groups and R¹, X¹, Q¹, G¹, G², G³, G⁴ and G⁵ have any of the meanings defined hereinbefore except that any functional group is protected if necessary, whereafter any protecting group that is present is removed by conventional means.

In *Reaction Scheme* 3, a suitable displaceable group L² in the compound of the Formula **VII** is, for example, halogeno or a sulfonyloxy group, for example fluoro, chloro, bromo, iodo, methylsulfonyloxy or toluene-4-sulfonyloxy group. A particular group L² is bromo, chloro or methylsulfonyloxy. The suitable displaceable groups L⁴ and L⁵ are as hereinbefore defined.

The reaction of a compound of the Formula **IIc** and a compound of the Formula **IId'** is conveniently carried out using analogous conditions to those discussed above for the reaction of a quinazoline of the Formula **IIc** and a compound of the Formula **IId.**

The reaction of a compound of the Formula **IIe** and a compound of the Formula **VII** is conveniently carried out using analogous conditions to those discussed below for Process (c).

A quinazoline of the Formula **IIc** may be obtained using conventional methods, for example, when R¹ is hydrogen, L⁴ is fluoro and L⁵ is halogeno, 5-fluoro-3,4-dihydroquinazolin-4-one may be reacted with a suitable halogenating agent such as thionyl chloride, phosphoryl chloride or a mixture of carbon tetrachloride and triphenylphosphine. The 5-fluoro-3,4-dihydroquinazoline starting material is commercially available or can be prepared using conventional methods, for example as described in J. Org. Chem. 1952, 17, 164-176.

Compounds of the Formula **IId** and **IId'** are commercially available compounds or they are known in the literature, or they can be can be prepared by standard processes known in the art. For example, compounds of the Formula **IId** or **IId'** may be prepared as illustrated in *Reaction Scheme 4:* 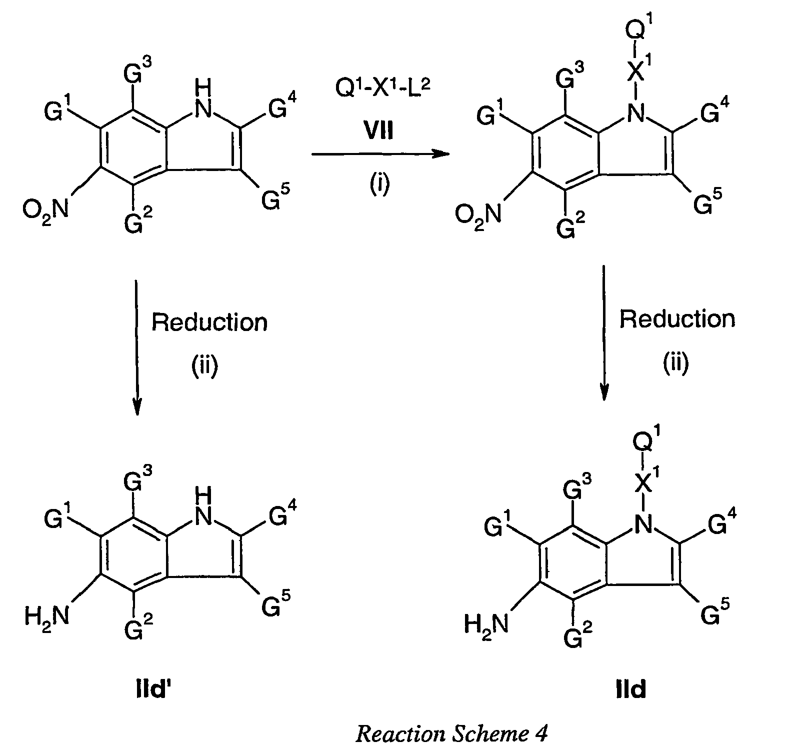 wherein L² is a suitable displaceable group as defined above and X¹, Q¹, G¹, G², G³, G⁴ and G⁵ have any of the meanings defined hereinbefore except that any functional group is protected if necessary, whereafter any protecting group that is present is removed by conventional means.

The reaction of step (i) in *Reaction Scheme 4*_is conveniently carried out using analogous conditions to those discussed below for Process (c).

The reduction in step (ii) in *Reaction Scheme 4*_may be conducted using conventional methods. For example, the reduction of the nitro group in step (ii) may be carried out under standard conditions, for example by catalytic hydrogenation over a platinum/carbon, palladium/carbon or nickel catalyst or a platinum (IV) oxide, treatment with a metal such as iron, titanium (III) chloride, tin (II) chloride or indium, or treatment with another suitable reducing agent such as sodium dithionite.

The quinazoline of the Formula **II** may alternatively be obtained a conventional procedure, for example as illustrated in *Reaction Scheme 5:* 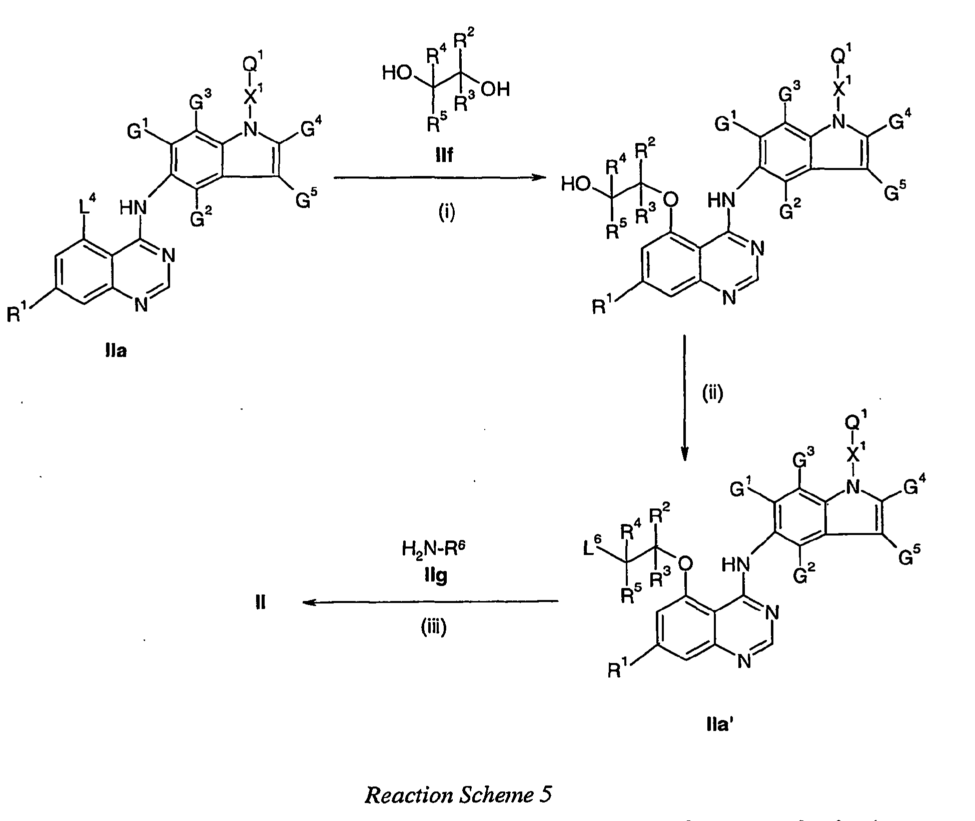 wherein L⁴ and L⁶ are suitable displaceable groups and R¹, R², R³, R⁴, R⁵, R⁶, X¹, Q¹, G¹, G², G³, G⁴ and G⁵ have any of the meanings defined hereinbefore except that any functional group is protected if necessary.

A suitable displaceable group L⁴ is as hereinbefore defined. For example, L⁴ may be halogeno, such as chloro or fluoro.

A suitable displaceable group L⁶ in the compound of the Formula **IIa'** is for example a halogeno or a sulfonyloxy group, for example a fluoro, chloro, methylsulfonyloxy or toluene-4-sulfonyloxy group. A particular group L⁶ is fluoro, chloro or methylsulfonyloxy, particularly chloro.

Step (i) of *Reaction Scheme* 5 may be conducted using analogous conditions to those used for the reaction of a compound of the Formula **IIa** and an alcohol of the Formula **IIb** or **IIb',** as discussed above.

Step (ii) of *Reaction Scheme* 5 may be conducted using a suitable conversion reaction. For example when L⁶ is chloro, step (ii) may be conducted using an appropriate chlorinating agent, such as thionyl chloride.

In step (iii) of *Reaction Scheme* 5, the reaction of the compound of the Formula **IIa'** with an amine of the Formula **IIg** may conveniently be carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or an alkali or alkaline earth metal carbonate such as sodium carbonate, potassium carbonate or caesium carbonate, calcium carbonate, or an alkali metal hydride such as sodium hydride. Alternatively, the reaction may use an excess of the amine of the Formula **IIg** in place of the aforementioned suitable base.

If necessary, the reaction of the compound of the Formula **IIa'** with an amine of the Formula **IIg** may conveniently be carried out in the presence of a suitable catalyst, for example tetrabutylammonium iodide.

The reaction of the compound of the Formula **IIa'** and the amine of the Formula **IIg** may conveniently be carried out in the presence of a suitable inert solvent or diluent, for example an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction may conveniently be carried out at a temperature in the range of, for example, from 25 to 150°C, conveniently at about 100°C.

Compounds of the Formula **IIa** may be obtained using conventional procedures, for example as discussed above.

Compounds of the Formulae **IIf** and **IIg** are commercially available compounds or they are known in the literature, or they can be prepared by standard processes known in the art.

### Process (b)

### Reaction Conditions for Process (b)

A suitable displaceable group L¹ in a compound of the Formula **IV** is for example a halogeno or a sulfonyloxy group, for example a fluoro, chloro, methylsulfonyloxy or toluene-4-sulfonyloxy group. A particular displaceable group L¹ is fluoro, chloro or methylsulfonyloxy, particularly chloro.

The reaction of the compound of the Formula **IV** with the amine of the Formula **V** may conveniently be carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or an alkali or alkaline earth metal carbonate such as sodium carbonate, potassium carbonate, cesium carbonate or calcium carbonate, or an alkali metal hydride such as sodium hydride. Alternatively, the reaction may use an excess of the amine of the Formula **V** in place of the aforementioned suitable base.

If necessary, the reaction may conveniently be carried out in the presence of a suitable catalyst, for example tetrabutylammonium iodide.

The reaction of the compound of the Formula **IV** and the amine of the Formula **V** is conveniently carried out in the presence of a suitable inert solvent or diluent, for example an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction may conveniently be carried out at a temperature in the range of, for example, from 25 to 150°C, conveniently at about 100°C.

### Preparation of Starting Materials for Process (b)

A quinazoline of the Formula **IV** may be prepared using conventional methods, for example, as discussed above.

Amines of the Formula **V** are commercially available compounds or they are known in the literature, or they can be can be prepared by standard processes known in the art.

### Process (c)

### Reaction Conditions for Process (c)

A suitable displaceable group L² in the compound of the Formula **VII** is, for example, halogeno or a sulfonyloxy group, for example fluoro, chloro, bromo, iodo, methylsulfonyloxy or toluene-4-sulfonyloxy group. A particular displaceable group L² is bromo, chloro or methylsulfonyloxy.

The reaction of a quinazoline of the Formula **VI** with a compound of the Formula **VII** is conveniently carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, such as sodium carbonate, potassium carbonate, cesium carbonate or calcium carbonate, or, for example, an alkali metal hydride, such as sodium hydride.

The reaction of a quinazoline of the Formula **VI** with a compound of the Formula **VII** is conveniently carried out in the presence of a suitable inert solvent or diluent, for example a halogenated solvent such as methylene chloride, chloroform or carbon tetrachloride, an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. Alternatively, the reaction may be conducted in the absence of an inert solvent or diluent. The reaction may conveniently be carried out at a temperature in the range of, for example, from 25 to 100°C, conveniently at or near ambient temperature.

### Preparation of Starting Materials for Process (c)

A quinazoline of the Formula **VI** may be prepared using conventional methods, for example, by reacting a compound of the Formula **VIa:** 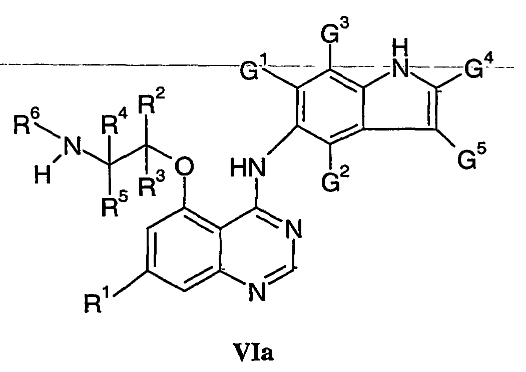 wherein R¹, R², R³, R⁴, R⁵, R⁶, G¹, G², G³, G⁴ and G⁵ are as hereinbefore defined except that any functional group is protected if necessary, with a carboxylic acid of the Formula **III,** or a reactive derivative thereof:

A-COOH **III**

wherein A has any of the meanings defined hereinbefore except that any functional group is protected if necessary and whereafter any protecting group that is present is removed by conventional means.

The reaction of a quinazoline of the Formula **VIa** and a compound of the Formula **III** is conveniently carried out using analogous conditions to those described above for Process (a).

Compounds of the Formula **VII** are commercially available compounds or they are known in the literature, or they can be can be prepared by standard processes known in the art.

### Process (d)

The reaction of the compounds of the Formula **VIII** and of the Formula **IX** is conveniently carried out using analogous conditions to those described above for the reaction of a quinazoline of the Formula **IIc** and a compound of the Formula **IId.**

### Reparation of Starting Materials for Process (d)

The quinazoline of the Formula **VIII** may be obtained by conventional procedures, as discussed above.

The compounds of the Formula **IX** are commercially available compounds or they are known in the literature, or they can be can be prepared by standard processes known in the art.

The quinazoline derivative of the Formula **I** may be obtained from the above processes in the form of the free base or alternatively it may be obtained in the form of a salt, for example an acid addition salt. When it is desired to obtain the free base from a salt of the quinazoline derivative of the Formula **I,** the salt may be treated with a suitable base, for example, an alkali or alkaline earth metal carbonate or hydroxide, for example sodium carbonate, potassium carbonate, calcium carbonate, sodium hydroxide or potassium hydroxide, or by treatment with ammonia for example using a methanolic ammonia solution such as 7N ammonia in methanol.

The protecting groups used in the processes above may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1 to 4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1 to 17 carbon atoms). Examples of carboxy protecting groups include straight or branched chain (1 to 12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

It will be appreciated that certain of the various ring substituents in the quinazoline derivatives of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group.

When a pharmaceutically acceptable salt of a quinazoline derivative of the Formula **I** is required, for example an acid-addition salt, it may be obtained by, for example, reaction of said quinazoline derivative with a suitable acid using a conventional procedure.

As mentioned hereinbefore some of the compounds according to the present invention may contain one or more chiral centers and may therefore exist as stereoisomers. Stereoisomers may be separated using conventional techniques, e.g. chromatography or fractional crystallisation. The enantiomers may be isolated by separation of a racemate for example by fractional crystallisation, resolution or HPLC. The diastereoisomers may be isolated by separation by virtue of the different physical properties of the diastereoisomers, for example, by fractional crystallisation, HPLC or flash chromatography. Alternatively particular stereoisomers may be made by chiral synthesis from chiral starting materials under conditions which will not cause racemisation or epimerisation, or by derivatisation, with a chiral reagent. When a specific stereoisomer is isolated it is suitably isolated substantially free for other stereoisomers, for example containing less than 20%, particularly less than 10% and more particularly less than 5% by weight of other stereoisomers.

In the section above relating to the preparation of the quinazoline derivative of the Formula **I**, the expression "inert solvent" refers to a solvent which does not react with the starting materials, reagents; intermediates or products in a manner which adversely affects the yield of the desired product.

Persons skilled in the art will appreciate that, in order to obtain quinazoline derivatives of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

Certain intermediates used in the processes described above are novel and form a further feature of the present invention. Accordingly there is provided a compound selected from a compound the Formulae **II, IV, VI** and **VIII** as hereinbefore defined, or a salt thereof. The intermediate may be in the form of a salt of the intermediate. Such salts need not be a pharmaceutically acceptable salt. For example it may be useful to prepare an intermediate in the form of a pharmaceutically non-acceptable salt if, for example, such salts are useful in the manufacture of a quinazoline derivative of the Formula **I**.

Particular intermediate compounds of the invention are, for example, one or more quinazoline derivatives of the Formula **II** selected from:
5-[(1R)-1-methyl-2-(methylamino)ethoxy]-N-[1-(pyridin-2-ylmethyl)-1H-indol-5-yl]quinazolin-4-amine; and
5-[(*R*)-2-(methylamino)propoxy]-*N*-[1-(pyridin-2-ylmethyl)-1*H*-indol-5-yl]quinazolin-4-amine
   or a salt thereof.

### Biological Assays

The inhibitory activities of compounds were assessed in non-cell based protein tyrosine kinase assays as well as in cell based proliferation assays before their in vivo activity was assessed in Xenograft studies.

### a) Protein Tyrosine Kinase phosphorylation Assays

This test measures the ability of a test compound to inhibit the phosphorylation of a tyrosine containing polypeptide substrate by EGFR, erbB2 and erbB4 tyrosine kinase enzyme.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM N-2-hydroxyethylpiperizine-N'-2-ethanesulfonic acid (HEPES) pH7.5, 150mM NaCl, 10% glycerol, 1% Triton X-100,1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 100µl phosphate buffered saline (PBS) solution and incubated at 4°C overnight). Plates were washed in 50mM HEPES pH 7.4 at room temperature to remove any excess unbound synthetic peptide. EGFR or erbB2 activities were assessed by incubation in peptide coated plates for 20 minutes at room temperature in 50mM HEPES pH 7.4 at room temperature, adenosine trisphosphate (ATP) at Km concentration for the respective enzyme, 10mM MnCl₂, 0.05mM Na₃VO₄, 0.1mM DL-dithiothreitol (DTT), 0.05% Triton X-100 with test compound in DMSO (final concentration of 2.5%). Reactions were terminated by the removal of the liquid components of the assay followed by washing of the plates with PBS-T (phosphate buffered saline with 0.05% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulfonate (6)] diammonium salt crystals (ABTS™ from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) EGFR driven KB cell proliferation assay

This assay measures the ability of a test compound to inhibit the proliferation of human tumour cell line, KB (obtained from the American Type Culture Collection (ATCC)).

KB cells were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks using Trypsin / ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulfoxide (DMSO) (0.1% final) before incubation for 4 days. Following the incubation period, cell numbers were determined by addition of 50µl of 3-(4,5-Dimethylthiazol-2-yl)-2,5-diphenyltetrazolium bromide (MTT) (stock 5mg/ml) for 2 hours. MTT solution was then tipped off, the plate gently tapped dry and the cells dissolved upon the addition of 100µl of DMSO.

Absorbance of the solubilised cells was read at 540nm using a Molecular Devices ThermoMax microplate reader. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) Clone 24 phospho-erbB2 cell assay

This immunofluorescence end point assay measures the ability of a test compound to inhibit the phosphorylation of erbB2 in a MCF7 (breast carcinoma) derived cell line which was generated by transfecting MCF7 cells with the full length erbB2 gene using standard methods to give a cell line that overexpresses full length wild type erbB2 protein (hereinafter 'Clone 24' cells).

Clone 24 cells were cultured in Growth Medium (phenol red free Dulbecco's modified Eagle's medium (DMEM) containing 10% foetal bovine serum, 2 mM glutamine and 1.2mg/ml G418) in a 7.5% CO₂ air incubator at 37°C. Cells were harvested from T75 stock flasks by washing once in PBS (phosphate buffered saline, pH7.4, Gibco No. 10010-015) and harvested using 2mls of Trypsin (1.25mg/ml) / ethylaminediaminetetraacetic acid (EDTA) (0.8mg/ml) solution. The cells were resuspended in Growth Medium. Cell density was measured using a haemocytometer and viability was calculated using Trypan Blue solution before being further diluted in Growth Medium and seeded at a density of 1x10⁴ cells per well (in 100µl) into clear bottomed 96 well plates (Packard, No. 6005182).

3 days later, Growth Medium was removed from the wells and replaced with 100ul Assay Medium (phenol red free DMEM, 2mM glutamine, 1.2mg/ml G418) either with or without erbB inhibitor compound. Plates were returned to the incubator for 4 hours and then 20µl of 20% formaldehyde solution in PBS was added to each well and the plate was left at room temperature for 30 minutes. This fixative solution was removed with a multichannel pipette, 100µl of PBS was added to each well and then removed with a multichannel pipette and then 50µl PBS was added to each well. Plates were then sealed and stored for up to 2 weeks at 4°C.

Immunostaining was performed at room temperature. Cells were washed once with 200µl PBS / Tween 20 (made by adding 1 sachet of PBS / Tween dry powder (Sigma, No. P3563) to 1L of double distilled H₂O) using a plate washer, then 100µl of 0.5% Triton X-100 /PBS was added to each well to permeabalise the cells. After 10 minutes, the plates were washed with 200µl PBS / Tween 20 and then 100µl Blocking Solution (5% Marvel dried skimmed milk (Nestle) in PBS) was added per well and the plates were incubated for 15 minutes. Following removal of the Blocking Solution with a plate washer, 30µl of rabbit polyclonal anti-phospho ErbB2 IgG antibody (epitope phospho-Tyr 1248, SantaCruz, No. SC-12352-R), diluted 1:250 in Blocking Solution, was added to each well and incubated for 2 hours. Then this primary antibody solution was removed from the wells using a plate washer followed by two 200µl PBS / Tween 20 washes using a plate washer. 100µl of Blocking solution was added per well and the plates were incubated for 10 minutes. Then 30µl of Alexa-Fluor 488 goat anti-rabbit IgG secondary antibody (Molecular Probes, No. A-11008), diluted 1:750 in Blocking Solution, was added to each well. From now onwards, wherever possible, plates were protected from light exposure, at this stage by sealing with black backing tape. The plates were incubated for 45 minutes and then the secondary antibody solution was removed from the wells followed by three 200µl PBS / Tween 20 washes using a plate washer. Then 50µl of PBS was added to each well and plates were resealed with black backing tape and stored at 4°C before analysis. Plates were analysed within six hours of completing the immunostaining.

The Fluorescence signal is each well was measured using an Acumen Explorer Instrument (Acumen Bioscience Ltd.), a plate reader that can be used to rapidly quantitate features of images generated by laser-scanning. The instrument was set to measure the number of fluorescent objects above a pre-set threshold value and this provided a measure of the phosphorylation status of erbB2 protein. Fluorescence dose response data obtained with each compound was exported into a suitable software package (such as Origin) to perform curve fitting analysis. Inhibition of erbB2 phosphorylation was expressed as an IC₅₀ value. This was determined by calculation of the concentration of compound that was required to give 50% inhibition of erbB2 phosphorylation signal.

### d) In vivo BT474C Xenograft assay

This assay measures the ability of a test compound to inhibit the growth of a specific variant of the BT-474 tumour cell line grown as a xenograft in Female Swiss athymic mice (Alderley Park, *nu*/*nu* genotype) (Baselga, J. et al. (1998) Cancer Research, 58, 2825-2831).

The BT-474 tumour cell line (human mammary carcinoma) was obtained from Dr Baselga (at Laboratorio Recerca Oncologica, Paseo Vall D'Hebron 119-129, Barcelona 08035, Spain). This cell line was subcloned and a certain population (hereinafter referred to as "BT474C") was obtained.

Female Swiss athymic (*nulnu* genotype) mice were bred and maintained in Alderley Park in negative pressure Isolators (PFI Systems Ltd.). Mice were housed in a barrier facility with 12 hour light/dark cycles and provided with sterilised food and water *ad libitum.* All procedures were performed on mice of at least 8 weeks of age. BT474C tumour cell xenografts were established in the hind flank of donor mice by sub-cutaneous injections of 1x10⁷ freshly cultured cells in 100µl of serum free media with 50% Matrigel per animal. Animals were supplemented with oestradiol benzoate (Mesalin, Intravet UK 0.2 mg/ml), 100µg/animal injected subcutaneously on the day before cell implant, with subsequent weekly boosts of 50µg/animal. On day 14 post-implant, mice were randomised into groups of 10 prior to the treatment with compound or vehicle control that was administered once daily at 0.1ml/10g body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students *t* test.

### e) BT474C Cell Proliferation Assay

BT474C cells are a sub-cloned population of *in vivo* competent cells, as discussed above.

The BT474C assay is a MTS (3-(4,5-dimethylthiazol-2-yl)-5-(3-carboxymethoxyphenyl)-2-(4-sulfophenyl)-2H- tetrazolium, inner salt - Promega G1111) endpoint-based cell proliferation assay, which measures the ability of a test compound to inhibit the proliferation of cells over a four-day period. Cells are grown to logarithmic phase in growth media (phenol red free Dulbecco's modified Eagle's medium (DMEM) containing 10% foetal bovine serum, 10% M1 supplement (AstraZeneca internal supply), 1% oxaloacetic acid in a 7.5% CO₂ air incubator at 37°C. Cells are harvested from stock flasks by washing once in PBS (phosphate buffered saline, pH7.4, Gibco No. 10010-015) and removed using 2mls of Trypsin (1.25mg/ml) / ethylaminediaminetetraacetic acid (EDTA) (0.8mg/ml) solution. The cells are re-suspended in assay media (phenol red free Dulbecco's modified Eagle's medium (DMEM) containing 10% charcoal/Dextran stripped foetal bovine serum, 10% M1 supplement, 1% oxaloacetic acid. Cell density is measured using a haemocytometer and viability is calculated using Trypan Blue solution before being further diluted in Assay Medium and seeded at a density of 1x10⁴ cells per well (in 100µl) into clear bottomed 96 well plates (Costar 3598). One extra plate is set up to act as a Day 0 control plate.

4 hours later, assay medium containing test compound, serially diluted in 100% DMSO (Sigma D5879), in the form of a dose response is added across the plate in triplicate. The Day 0 plate is treated with MTS solution (Tetrazolium compound - made from MTS powder in a Phenazine ethosulfate (PES - Sigma P4544)/PBS) and incubated for 2 hours before the reaction is stopped by the addition of 10% SDS. The plate is read at 490nm on a spectrophotometer.

Assay plates are left at 37°C for 4 days and then treated with MTS solution (as above), which is converted to a soluble formazan product by active cells. After incubating the plates for 2 hours the reaction is stopped by the addition of 10% SDS (Sodium dodecyl sulphate) and the plates are read at 490nm on a spectrophotometer giving absorbance values relative to the concentration of converted dye.

Absorbance dose response data obtained with each compound is exported into a suitable software package (such as Origin) to perform curve-fitting analysis. Inhibition of BT474C cell proliferation is expressed as an IC₅₀ value (calculated as GI50 by use of a log/lin plot - analyzing data above the day 0 absorbance values). This is determined by calculation of the concentration of compound that is required to give 50% inhibition of cell proliferation.

### f) hERG-encoded Potassium Channel Inhibition Assay

### Cell culture for IonWorks™ HT:

The hERG-expressing Chinese hamster ovary K1 (CHO) cells described by Persson *et al.* (Persson, F., Carlsson, L., Duker, G., and Jacobson, I., Blocking characteristics of hERG, hNav1.5, and hKvLQT1/hminK after administration of the novel anti-arrhythmic compound AZD7009., J Cardiovasc.Electrophysiol., 16, 329-341.2005) were grown to semi-confluence at 37°C in a humidified environment (5%CO₂) in F-12 Ham medium containing L-glutamine, 10% foetal calf serum (FCS) and 0.6 mg/ml hygromycin (all Sigma). Prior to use, the monolayer was washed using a pre-warmed (37°C) 3ml aliquot of Versene 1:5,000 (Invitrogen). After aspiration of this solution the flask was incubated at 37°C in an incubator with a further 2 ml of Versene 1:5,000 for a period of 6 minutes. Cells were then detached from the bottom of the flask by gentle tapping and 10 ml of Dulbecco's-PBS containing calcium (0.9 mM) and magnesium (0.5 mM) (PBS; Invitrogen) was then added to the flask and aspirated into a 15 ml centrifuge tube prior to centrifugation (50 g, for 4 minutes). The resulting supernatant was discarded and the pellet gently re-suspended in 3 ml of PBS. A 0.5 ml aliquot of cell suspension was removed to determine viable cell number based on trypan blue exclusion (Cedex; Innovatis) and the cell re-suspension volume adjusted with PBS to give the desired final cell concentration. CHO-Kv1.5 cells, which were used to adjust the voltage offset on IonWorks™ HT, were maintained and prepared for use in the same way.

### IonWorks™ HT electrophysiology:

The principles and operation of this device have been described by Schroeder *et al.* (Schroeder, K., Neagle, B., Trezise, D. J., and Worley, J., Ionworks HT: a new high-throughput electrophysiology measurement platform, J Biomol Screen, **8**, 50-64, 2003). Briefly, the technology is based on a 384-well plate (PatchPlate^{™}) in which a recording is attempted in each well by using suction to position and hold a cell on a small hole separating two isolated fluid chambers. Once sealing has taken place, the solution on the underside of the PatchPlate^{™} is changed to one containing amphotericin B. This permeablises the patch of cell membrane covering the hole in each well and in effect allows a perforated, whole-cell patch clamp recording to be made.

IonWorks™ HT (a beta-test machine from Essen Instruments) was operated at room temperature (∼21°C) in the following way. The reservoir in the "Buffer" position was loaded with 4 ml of PBS and that in the "Cells" position with the CHO-hERG cell suspension described above. A 96-well plate (V-bottom, Greiner Bio-one) containing the compounds to be tested (at 3X their final test concentration) was placed in the "Plate 1" position and a PatchPlate™ was clamped into the PatchPlate™ station. Each compound plate was laid-out in 12 columns to enable ten, 8-point concentration-effect curves to be constructed; the remaining two columns on the plate were taken up with vehicle (final concentration 0.33% DMSO), to define the assay baseline, and a supra-maximal blocking concentration of cisapride (final concentration 10 µM), to define the 100% inhibition level. The fluidics-head (F-Head) of IonWorks™ HT then added 3.5 µl of PBS to each well of the PatchPlate™ and its underside was perfused with "internal" solution that had the following composition (in mM): K-Gluconate 100, KCl 40, MgCl₂ 3.2, EGTA 3 and HEPES 5 (all Sigma) (pH 7.25-7.30 using 10 M KOH). After priming and de-bubbling, the electronics-head (E-head) then moved round the PatchPlate™ performing a hole test (i.e. applying a voltage pulse to determine whether the hole in each well was open). The F-head then dispensed 3.5 µl of the cell suspension described above into each well of the PatchPlate™ and the cells were given 200 seconds to reach and seal to the hole in each well. Following this, the E-head moved round the PatchPlate™ to determine the seal resistance obtained in each well. Next, the solution on the underside of the PatchPlate™ was changed to "access" solution that had the following composition (in mM): KCl 140, EGTA 1, MgCl₂ 1 and HEPES 20 (pH 7.25-7.30 using 10 M KOH) plus 100 µg/ml of amphotericin B (all Sigma). After allowing 9 minutes for patch perforation to take place, the E-head moved round the PatchPlate™ 48 wells at a time to obtain pre-compound hERG current measurements. The F-head then added 3.5 µl of solution from each well of the compound plate to 4 wells on the PatchPlate™ (the final DMSO concentration was 0.33% in every well). This was achieved by moving from the most dilute to the most concentrated well of the compound plate to minimise the impact of any compound carry-over. After approximately three and a half minutes incubation, the E-head then moved around all 384-wells of the PatchPlate™ to obtain post-compound hERG current measurements. In this way, non-cumulative concentration-effect curves could be produced where, providing the acceptance criteria were achieved in a sufficient percentage of wells (see below), the effect of each concentration of test compound was based on recording from between 1 and 4 cells.

The pre- and post-compound hERG current was evoked by a single voltage pulse consisting of a 20 s period holding at -70 mV, a 160 ms step to -60 mV (to obtain an estimate of leak), a 100 ms step back to -70 mV, a 1 s step to +40 mV, a 2 s step to -30 mV and finally a 500 ms step to -70mV. In between the pre- and post-compound voltage pulses there was no clamping of the membrane potential. Currents were leak-subtracted based on the estimate of current evoked during the +10mV step at the start of the voltage pulse protocol. The current signal was sampled at 2.5k Hz.

Pre- and post-scan hERG current magnitude was measured automatically from the leak subtracted traces by the IonWorks™ HT software by taking a 40ms average of the current during the initial holding period at -70mV (baseline current) and subtracting this from the peak of the tail current response. The acceptance criteria for the currents evoked in each well were: pre-scan seal resistance >60 MΩ, pre-scan hERG tail current amplitudes >150 pA; post-scan seal resistance >60 MΩ. The degree of inhibition of the hERG current was assessed by dividing the post-scan hERG current by the respective pre-scan hERG current for each well.

Although the pharmacological properties of the quinazoline derivatives of the Formula **I** vary with structural change as expected, in general activity possessed by quinazoline derivatives of the Formula **I**, may be demonstrated at the following concentrations or doses in one or more of the above tests (a), (b), (c), (d) and (e):-
Test (a):- IC₅₀ in the range, for example, 0.001 - 1 µM;
Test (b):- IC₅₀ in the range, for example, 0.001 - 5 µM;
Test (c):- IC₅₀ in the range, for example, 0.001 - 5 µM;
Test (d):- activity in the range, for example, 1-200 mg/kg/day;
Test (e):- IC₅₀ in the range, for example, 0.001 - 1 µM;

No physiologically unacceptable toxicity was observed in Test (d) at the effective dose for quinazoline derivatives tested of the present invention. Test (f) shows a safe margin between target and hERG activity, suggesting the unlikelihood of arrhythmia caused by inhibition of the hERG channel. Accordingly no untoward toxicological effects are expected when a quinazoline derivative of the Formula **I**, or a pharmaceutically acceptable salt thereof, as defined hereinbefore is administered at the dosage ranges defined hereinafter.

By way of example, Table A illustrates the activity of a representative quinazoline derivative according to the invention. Column 2 of Table A shows IC₅₀ data from Test (a) for the inhibition of EGFR tyrosine kinase protein phosphorylation; column 3 shows IC₅₀ data from Test (a) for the inhibition of erbB2 tyrosine kinase protein phosphorylation; and column 4 shows IC₅₀ data for inhibition of phosphorylation of erbB2 in a MCF7 derived cell line in Test (c) described above:

**Table A**

| **Example Number** | **IC₅₀ (µM)** **Test (a):** **Inhibition of** **EGFR tyrosine** **kinase protein** **phosphorylation** | **IC₅₀ (µM)** **Test (a):** **Inhibition of** **erbB2 tyrosine** **kinase protein** **phosphorylation** | **IC₅₀ (µM)** **Test (c):** **Inhibition of** **erbB2 tyrosine** **kinase protein** **phosphorylation** |
|---|---|---|---|
| 1 | 0.78 | 0.071 | 1.007 |
| 2 | 0.063 | 0.005 | 0.002 |

According to a further aspect of the invention there is provided a pharmaceutical composition which comprises a quinazoline derivative of the Formula **I**, or a pharmaceutically acceptable thereof, as defined hereinbefore in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a quinazoline derivative of the Formula **I** will naturally vary according to the nature and severity of the conditions, the age and sex of the animal or patient and the route of administration, according to well known principles of medicine.

In using a quinazoline derivative of the Formula **I** for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a quinazoline derivative of this invention.

We have found that the quinazoline derivatives of the present invention possess anti-proliferative properties such as anti-cancer properties that are believed to arise from their erbB, particularly EGF and more particularly erbB2 receptor tyrosine kinase inhibitory activity. Furthermore, certain of the quinazoline derivatives according to the present invention possess substantially better potency against the erbB2 receptor tyrosine kinase, than against other tyrosine kinases enzymes, such as EGFR tyrosine kinase. Such quinazoline derivatives possess sufficient potency against the erbB2 receptor tyrosine kinase that they may be used in an amount sufficient to inhibit erbB2 receptor tyrosine kinase whilst demonstrating little, or significantly lower, activity against other tyrosine kinases such as EGFR. Such quinazoline derivatives are likely to be useful for the selective inhibition of erbB2 receptor tyrosine kinase and are likely to be useful for the effective treatment of, for example erbB2 driven tumours.

Accordingly, the quinazoline derivatives of the present invention are expected to be useful in the treatment of diseases or medical conditions mediated alone or in part by and erbB, particularly erbB2 receptor tyrosine kinases, i.e. the compounds may be used to produce an erbB, particularly an erbB2, receptor tyrosine kinase inhibitory effect in a warm-blooded animal in need of such treatment. Thus the quinazoline derivatives of the present invention provide a method for the treatment of malignant cells characterised by inhibition of the erbB, particularly the erbB2, receptor tyrosine kinase. Particularly the quinazoline derivatives of the invention may be used to produce an anti-proliferative and/or pro-apoptotic and/or anti-invasive effect mediated alone or in part by the inhibition of erbB, particularly erbB2, receptor tyrosine kinases. Particularly, the quinazoline derivatives of the present invention are expected to be useful in the prevention or treatment of those tumours that are sensitive to inhibition of an erbB, particularly the erbB2, receptor tyrosine kinase that are involved in the signal transduction steps which drive proliferation and survival of these tumour cells. Accordingly the quinazoline derivatives of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, erbB, more particularly erbB2, receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours.

According to this aspect of the invention there is provided a quinazoline derivative of the Formula **I**, or a pharmaceutically acceptable salt thereof, for use as a medicament.

Thus according to this aspect of the invention there is provided the use of a quinazoline derivative of the Formula **I**, or a pharmaceutically acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatment may comprise administering to said animal an effective amount of a quinazoline derivative of the Formula **I**, or a pharmaceutically acceptable salt thereof, as hereinbefore defined.

According to a further aspect of the invention there is provided a quinazoline derivative of the Formula **I**, or a pharmaceutically acceptable salt thereof, for use in the production of an anti-proliferative effect in a warm-blooded animal such as man.

According to a further aspect of the invention there is provided the use of a quinazoline derivative of the Formula **I**, or a pharmaceutically acceptable salt thereof, as defined hereinbefore in the manufacture of a medicament for use in the production of an anti-proliferative effect which effect is produced alone or in part by inhibiting erbB2 receptor tyrosine kinase in a warm-blooded animal such as man.

A method for producing an anti-proliferative effect which effect is produced alone or in part by inhibiting erbB2 receptors tyrosine kinase in a warm-blooded animal, such as man, in need of such treatments may comprise administering to said animal an effective amount of a quinazoline derivative of the Formula **I**, or a pharmaceutically acceptable salt thereof, as hereinbefore defined.

According to a further aspect of the invention there is provided a quinazoline derivative of the Formula **I**, or a pharmaceutically acceptable salt thereof, for use in the production of an anti-proliferative effect which effect is produced alone or in part by inhibiting erbB2 receptor tyrosine kinase in a warm-blooded animal such as man.

According to a further aspect of the present invention there is provided the use of a quinazoline derivative of the Formula **I**, or a pharmaceutically acceptable salt thereof, as defined hereinbefore in the manufacture of a medicament for use in the treatment of a disease or medical condition (for example a cancer as mentioned herein) mediated alone or in part by erbB, particularly erbB2, receptor tyrosine kinase.

A method for treating a disease or medical condition (for example a cancer as mentioned herein) mediated alone or in part by erbB, particularly erbB2, receptor tyrosine kinase in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in the treatment of a disease or medical condition (for example a cancer as mentioned herein) mediated alone or in part by erbB, particularly erbB2, receptor tyrosine kinase.

According to a further aspect of the invention there is provided the use of a quinazoline derivative of the Formula **I**, or a pharmaceutically acceptable salt thereof, as defined hereinbefore in the manufacture of a medicament for use in the prevention or treatment of those tumours which are sensitive to inhibition of one or more erbB receptor tyrosine kinases, such as EGF and/or erbB2 and/or erbB4 (especially erbB2) receptor tyrosine kinases, that are involved in the signal transduction steps which lead to the proliferation of tumour cells. A method for the prevention or treatment of those tumours which are sensitive to inhibition of one or more erbB receptor tyrosine kinases, such as EGF and/or erbB2 and/or erbB4 (especially erbB2) receptor tyrosine kinase, that are involved in the signal transduction steps which lead to the proliferation and/or survival of tumour cells in a warm-blooded animal, such as man, in need of such treatment, may comprise administering to said animal an effective amount of a quinazoline derivative of the Formula **I,** or a pharmaceutically acceptable salt thereof, as defined hereinbefore.

According to a further aspect of the invention there is provided a quinazoline derivative of the Formula **I,** or a pharmaceutically acceptable salt thereof, for use in the prevention or treatment of those tumours which are sensitive to inhibition of one or more erbB receptor tyrosine kinases, such as EGF and/or erbB2 and/or erbB24 (especially erbB2) receptor tyrosine kinase, that are involved in the signal transduction steps which lead to the proliferation and/or survival of tumour cells.

According to a further aspect of the invention there is provided the use of a quinazoline derivative of the Formula **I**, or a pharmaceutically acceptable salt thereof, as defined hereinbefore in the manufacture of a medicament for use in providing an EGF and/or erbB2 and/or erbB4 (especially erbB22) receptor tyrosine kinase inhibitory effect.

A method for providing an EGF and/or erbB2 and/or erbB4 (especially erbB2) receptor tyrosine kinase inhibitory effect in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in providing an EGF and/or erbB2 and/or erbB4 (especially erbB2) receptor tyrosine kinase inhibitory effect.

According to a further aspect of the invention there is provided the use of a quinazoline derivative of the Formula **I**, or a pharmaceutically acceptable salt thereof, as defined hereinbefore in the manufacture of a medicament for use in providing a selective erbB2 kinase inhibitory effect.

A method for providing a selective erbB2 kinase inhibitory effect in a warm-blooded animal, such as man, in need of such treatment may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I,** or a pharmaceutically acceptable salt thereof, for use in providing a selective erbB2 kinase inhibitory effect.

By "a selective erbB2 kinase inhibitory effect" is meant that the quinazoline derivative of the Formula **I** is more potent against erbB2 receptor tyrosine kinase than it is against other kinases. In particular some of the compounds according to the invention are more potent against erbB2 receptor kinase than it is against other tyrosine kinases such as other erbB receptor tyrosine kinases, particularly EGFR tyrosine kinase. For example a selective erbB2 kinase inhibitor according to the invention is at least 5 times, preferably at least 10 times more potent against erbB2 receptor tyrosine kinase than it is against EGFR tyrosine kinase, as determined from the relative IC₅₀ values in suitable assays (for example the by comparing the IC₅₀ value from the Clone 24 phospho-erbB2 cell assay (a measure of the erbB2 tyrosine kinase inhibitory activity in cells) with the IC₅₀ from the KB cell assay (a measure of the EGFR tyrosine kinase inhibitory activity in cells) for a given test compound as described above).

According to a further aspect of the present invention there is provided the use of a quinazoline derivative of the Formula **I**, or a pharmaceutically acceptable salt thereof, as defined hereinbefore in the manufacture of a medicament for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

A method for treating a cancer, for example a cancer selected from selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

As mentioned above the size of the dose required for the therapeutic or prophlyactic treatment of a particular disease will necessarily be varied depending upon, amongst other things, the host treated, the route of administration and the severity of the illness being treated.

The anti-proliferative treatment defined hereinbefore may be applied as a sole therapy or may involve, in addition to the compound of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of
   5α-reductase such as finasteride;
(iii) anti-invasion agents (for example c-Src kinase family inhibitors like 4-(6-chloro-2,3-methylenedioxyanilino)-7-[2-(4-methylgiperazin-1-yl)ethoxy]-5-tetrahydropyran-4-yloxyquinazoline (AZD0530; International Patent Application WO 01/94341) and *N-*(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661), and metalloproteinase inhibitors like marimastat, inhibitors of urokinase plasminogen activator receptor function or antibodies to Heparanase);
(iv) inhibitors of growth factor function: for example such inhibitors include growth factor antibodies and growth factor receptor antibodies (for example the anti-erbB2 antibody trastuzumab [Herceptin™] and the anti-erbB1 antibody cetuximab [Erbitux, C225]); such inhibitors also include tyrosine kinase inhibitors, for example inhibitors of the epidermal growth factor family (for example EGFR family tyrosine kinase inhibitors such as *N-*(3-chloro-4-fluorophenyl)-7-methoxy-6-(3-morpholinopropoxy)quinazolin-4-amine (gefitinib, ZD1839), *N-*(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (CI 1033), erbB2 tyrosine kinase inhibitors such as lapatinib, inhibitors of the hepatocyte growth factor family, inhibitors of the platelet-derived growth factor family such as imatinib, inhibitors of serine/threonine kinases (for example Ras/Raf signalling inhibitors such as farnesyl transferase inhibitors, for example sorafenib (BAY 43-9006)), inhibitors of cell signalling through MEK and/or AKT kinases, inhibitors of the hepatocyte growth factor family, c-kit inhibitors, abl kinase inhibitors, IGF receptor (insulin-like growth factor) kinase inhibitors; aurora kinase inhibitors (for example AZD1152, PH739358, VX-680, MLN8054, R763, MP235, MP529, VX-528 and AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin™) and VEGF receptor tyrosine kinase inhibitors such as 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalanib (PTK787; WO 98/35985) and SU11248 (sunitinib; WO 01/60814), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the quinazoline derivatives of this invention within the dosage range described hereinbefore and the other pharmaceutically-active agent within its approved dosage range.

According to this aspect of the invention there is provided a pharmaceutical product comprising a quinazoline derivative of the Formula **I** as defined hereinbefore and an additional anti-tumour agent as defined hereinbefore for the conjoint treatment of cancer.

Although the quinazoline derivatives of the Formula **I** are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests and in the search for new pharmacological agents.

The invention will now be illustrated by the following non-limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30 mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulfoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms;
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulfur atom have not been resolved;
(xii) where a synthesis is described as being analogous to that described in a previous example the amounts used are the millimolar ratio equivalents to those used in the previous example;
(xiii) all microwave reactions were carried out in a CEM Discover™ microwave synthesisor;
(xiv) preparative high performance liquid chromatography (HPLC) was performed on a Gilson instrument using the following conditions:

| | |
|---|---|
| Column: | 21 mm x 10 cm Hichrom RPB |
| Solvent A: | Water + 0.1 % trifluoroacetic acid, |
| Solvent B: | Acetonitrile + 0.1% trifluoroacetic acid |
| Flow rate: | 18 ml / min |
| Run time: | 15 minutes with a 10 minute gradient from 5-95% B |
| Wavelength: | 254 nm, bandwidth 10 nm |
| Injection volume | 2.0-4.0 ml; and |

(xv) the following abbreviations have been used:
- HATU: O-(7-azabenzotriazol-1-yl)-N,N,N',N'-tetramethyluronium hexafluoro-phosphate; and
- THF: tetrahydrofuran;
- DMF: *N,N*-dimethylformamide;
- DMA: *N,N*-dimethylacetamide;
- DCM: dichloromethane;
- DMSO: dimethylsulfoxide;
- IPA: Isopropyl alcohol;
- ether: diethyl ether;
- TFA: trifluoroacetic acid.

### Example 1

### 2-Hydroxy-N-methyl-N-{(2R)-2-[(4-{[1-(pyridin-2-ylmethyl)-1H-indol-5-yl]amino}quinazolin-5-yl)oxy]propyl}acetamide

To a stirred solution of 5-[(1R)-1-methyl-2-(methylamino)ethoxy]-N-[1-(pyridin-2-ylmethyl)-1H-indol-5-yl]quinazolin-4-amine (204mg, 0.47mM and triethylamine (71mg, 0.71mM) in dichloromethane (5ml) at 0-4°C was added drop-wise, acetoxyacetyl chloride (71mg, 0.52mM). The solution was allowed to warm to ambient temperature and was stirred for 30 minutes. The solution was diluted with DCM, washed with aqueous Na₂CO₃, dried over anhydrous Na₂SO₄ and evaporated to a gum.

The gum was dissolved in 7.0M NH₃ / methanol (10ml) and stirred for 18 hours. The solvent was evaporated and the title compound was isolated by chromatography (silica, 5% methanol / DCM) to give an amorphous solid on trituration with ether (143mg, 61%); NMR spectrum (400MHz, 373°K) 1.47 (d, 3H), 2.98 (s, 3H), 3.60 (dd, 1H), 3.97 (bm, 1H), 4.08 (bm, 3H), 5.12 (m, 1H), 5.49 (s, 2H), 6.52 (d, 1H), 7.07 (d, 1H), 7.21 (d, 1H), 7.26 (dd, 1H), 7.32 (d, 1H), 7.40 (m, 3H), 7.68 (m, 2H), 8.05 (bm, 1H), 8.41 (s, 1H), 8.54 (d, 1H), 9.91 (s, 1H); Mass spectrum MH⁺ 497.

The 5-[(1R)-1-methyl-2-(methylamino)ethoxy]-N-[1-(pyridin-2-ylmethyl)-1H-indol-5-yl]quinazolin-4-amine used as starting material was prepared as follows:

(2*R*)-2-methyloxirane (13.76 g) was added to a suspension of *N-*methylprop-2-en-1-amine (25 ml) and ytterbium(III) trifluoromethanesulfonate (100 mg) in dioxane (100 ml) and heated to 140°C for 1 hour under microwave irradiation. The solution was concentrated *in vacuo* and the residue partitioned between water (100 ml) and ethyl acetate (200 ml). The organic extract was dried and solvent removed *in vacuo* to give (2R)-1-[allyl(methyl)amino]propan-2-ol as a yellow oil (8.8 g, 29%); NMR spectrum (CDCl₃) 1.20 (d, 3H), 2.33 (s, 3H), 2.27 - 2.46 (m, 2H), 3.05 (m, 1H), 3.23 (m, 1H), 3.88 (m, 1H), 5.19 - 5.29 (m, 2H), 5.90 (m, 1H); Mass spectrum M⁺ 129.

DMF (0.2 ml) was added to a suspension of 5-fluoro-3,4-dihydro-3H-quinazolin-4-one (1.64 g) in thionyl chloride (10 ml) and the mixture was stirred and heated at 80°C for 6 hours. Volatile material was removed by evaporation and the residue was azeotroped with toluene (20 ml). The resulting solid was added portion-wise to a vigorously stirred mixture of saturated sodium bicarbonate (50 ml), crushed ice (50 g) and DCM (50 ml) such that the temperature was kept below 5°C. The organic phase was separated, dried and concentrated to give 4-chloro-5-fluoroquinazoline as a solid (1.82 g, 99%) which was used without purification; NMR spectrum (CDCl₃) 7.35 - 7.45 (m, 1H), 7.85 - 7.95 (m, 2H), 9.0 (s, 1H).

A stirred partial solution of 4-chloro-5-fluoroquinazoline (10.77g, 59mM) and 5-aminoindole (7.80g, 59mM) in isopropanol (200ml) was heated under reflux for 4 hours. On cooling to ambient temperature, the product hydrochloride salt was filtered off and washed with isopropanol and ether. The salt was heated with water / ethanol and the partial solution was basified with aqueous ammonia. The precipitated 5-fluoro-N-1H-indol-5-ylquinazolin-4-amine was filtered off and washed with water (15.46g, 94%); NMR spectrum 6.42 (s, 1H), 7.29 (dd, 1H), 7.38 (m, 3H), 7.58 (d, 1H), 7.80 (m, 1H), 7.89 (s, 1H), 8.48 (s, 1H), 9.07 (d, 1H), 11.08 (s, 1H); Mass spectrum MH⁺ 279.

Sodium hydride (60% dispersion in mineral oil, 1.26g, 31.5mM) was added portion-wise to a stirred partial solution of 5-fluoro-N-1H-indol-5-ylquinazolin-4-amine (4.17g, 15mM) and 2-picolyl chloride hydrochloride (2.58, 15.75mM) in DMF (75ml). The reaction mixture was maintained at ambient temperature by slight cooling and stirred for 18 hours. The reaction mixture was quenched by addition of saturated aqueous ammonium chloride solution (5ml) and evaporated under hi-vacuum. The residue was partitioned between 2.5M aqueous NaOH and DCM and the organic phase was dried over anhydrous Na₂SO₄ and evaporated. The product was purified by chromatography (2%methanol / ethyl acetate) and crystallized on trituration with ether to give 5-fluoro-N-[1-(pyridin-2-ylmethyl)-1H-indol-5-yl]quinazolin-4-amine (1.34g, 24%); NMR spectrum 5.52 (s, 2H), 6.52 (d, 1H), 6.98 (d, 1H), 7.27 (m, 2H), 7.40 (m, 2H), 7.52 (d, 1H), 7.58 (d,1H), 7.70 (m, 1H), 7.80 (m, 1H), 7.90 (s, 1H), 8.47 (s, 1H), 8.55 (d, 1H), 9.07 (d, 1H); Mass spectrum MH⁺ 370.

Sodium hydride (60% dispersion in mineral oil, 100mg, 2.5mM) was suspended in stirred dry THF (5ml) and (2*R*)-1-[allyl(methyl)amino]propan-2-ol (323mg, 2.5mM) was added dropwise. After stirring for 5 to 10 minutes, 5-fluoro-N-[1-(pyridin-2-ylmethyl)-1H-indol-5-yl]quinazolin-4-amine (369mg, 1.0mM) was added and the mixture was heated at 130°C for 30 minutes in a microwave reactor. The reaction mixture was quenched by addition of saturated aqueous ammonium chloride solution (1ml) and partitioned between 2.5M aqueous NaOH and DCM. The organic phase was dried over anhydrous Na₂SO₄ and evaporated to give 5-{(1R)-2-[allyl(methyl)amino]-1-methylethoxy}-N-[1-(pyridin-2-ylmethyl)-1H-indol-5-yl]quinazolin-4-amine as a gum (448mg, 94%) which was used without further purification; Mass spectrum MH⁺ 479.

A stirred mixture of 5-{(1R)-2-[allyl(methyl)amino]-1-methylethoxy}-N-[1-(pyridin-2-ylmethyl)-1H-indol-5-yl]quinazolin-4-amine (448mg, 0.94mM) and chlorotris(triphenylphosphine)rhodium(70mg, 0.075mM) in MeCN / water 5:1 (6ml) was heated at 110°C for 20 minutes in a microwave reactor. The solvent was evaporated and the residue was partitioned between water and DCM. The organic phase was dried over anhydrous Na₂SO₄ and evaporated. The product was isolated by chromatography (silica, 2-10% ammonia- MeOH / DCM) and crystallized on trituration with ether to give 5-[(1R)-1-methyl-2-(methylamino)ethoxy]-N-[1-(pyridin-2-ylmethyl)-1H-indol-5-yl]quinazolin-4-amine, (273mg, 66%); NMR spectrum 1.43 (d, 3H), 2.13 (broad s, 1H), 2.33 (s, 3H), 2.89 (m, 2H), 4.89 (m, 1H), 5.50 (s, 2H), 6.50 (d, 1H), 6.96 (d, 1H), 7.14 (d, 1H), 7.25 (m, 2H), 7.38 (m, 2H), 7.50 (d, 1H), 7.68 (m, 2H), 8.13 (s, 1H), 8.40 (s, 1H), 8.53 (d, 1H), 10.52 (s, 1H); Mass spectrum MH⁺ 439.

### Example 2

### 2-Hydroxy-N-methyl-N-{(R)-1-methyl-2-[4-(1-pyridin-2-ylmethyl-1H-indol-5-ylamino)-quinazolin-5-yloxy]-ethyl}-acetamide

To a stirred solution of 5-[(*R*)-2-(methylamino)propoxy]-*N*-[1-(pyridin-2-ylmethyl)-1*H*-indol-5-yl]quinazolin-4-amine (306mg, 0.70mM), glycolic acid (64mg, 0.84mM) and diisopropylethylamine (226mg, 1.755mM) in DMF (8ml) at ambient temperature was added, portion-wise, HATU (319mg, 0.84mM). The solution was stirred at ambient temperature overnight. The solution was passed through an SCX-2 cartridge eluting first with methanol, then with 1% NH₃ / methanol solution. The latter fractions were combined and evaporated to afford a golden brown oil which was purified by chromatography (1-10% methanol / DCM) and crystallised on trituration with ether to give the title compound (221mg, 64%); NMR spectrum (400MHz, 373°K) 1.27 (d, 3H), 2.85 (s, 3H), 3.88 - 3.92 (m, 1H), 3.96 - 4.02 (m, 2H), 4.34 - 4.39 (m, 1H), 4.47 - 4.51 (m, 1H), 4.90 (bs, 1H), 5.48 (s, 2H), 6.50 - 6.51 (m, 1H), 7.06 (d, 1H), 7.18 (d, 1H), 7.24 - 7.30 (m, 2H), 7.32 - 7.35 (m, 1H), 7.39 - 7.44 (m, 2H), 7.66 - 7.72 (m, 2H), 7.89 - 7.91 (m, 1H), 8.38 (s, 1H), 8.53 - 8.55 (m, 1H), 9.62 (bs, 1H); Mass spectrum MH⁺ 497.

The 5-[(*R*)-2-(methylamino)propoxy]-*N*-[1-(pyridin-2-ylmethyl)-1*H*-indol-5-yl]quinazolin-4-amine used as starting material was prepared substantially as described in Example 1 (preparation of starting materials) using the starting materials 5-fluoro-N-[1-(pyridin-2-ylmethyl)-1H-indol-5-yl]quinazolin-4-amine and (R)-2-methylamino-propan-1-ol (prepared as described in Becker et al., J. Chem. Soc. 1957, 858). The crude material was purified by chromatography (1-10% methanol / DCM) to give 5-[(R)-2-(methylamino)propoxy]-N-[1-(pyridin-2-ylmethyl)-1H-indol-5-yl]quinazolin-4-amine in 80% yield; NMR spectrum (400 MHz) 1.18 (d, 3H), 2.36 (s, 3H), 3.05 - 3.12 (m, 1H), 4.12 - 4.15 (m, 1H), 4.27 - 4.30 (m, 1H), 5.52 (s, 2H), 6.52 (d, 1H), 7.01 (d, 1H), 7.10 (d, 1H), 7.27 - 7.31 (m, 2H), 7.41 (d, 1H), 7.46- 7.52 (m, 2H), 7.67 - 7.75 (m, 2H), 8.15 (d, 1H), 8.43 (s, 1H), 8.55 - 8.56 (m, 1H), 10.56 (s, 1H); Mass spectrum MH⁺ 439.

## Claims

1. A quinazoline derivative of the Formula **I**: 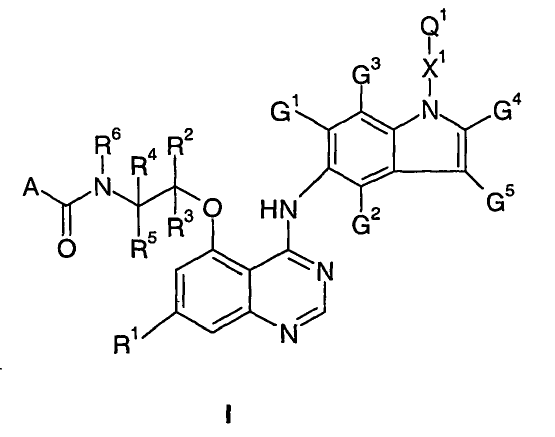 wherein:
**R¹** is selected from hydrogen, hydroxy, (1-4C)alkoxy and (1-4C)alkoxy(1-4C)alkoxy;
**G¹, G², G³, G⁴ and G⁵** are each, independently, selected from hydrogen and halogeno;
**X¹** is selected from SO₂, CO, SO₂N(R⁷) and C(R⁷)₂, wherein each R⁷ is, independently, selected from hydrogen and (1-4C)alkyl;
**Q¹** is aryl or heteroaryl, which aryl or heteroaryl group optionally bears one or more substituents independently selected from halogeno, cyano and (1-4C)alkoxy;
**R², R³, R⁴ and R⁵** are each, independently, selected from hydrogen and (1-4C)alkyl, or
R² and R³ together with the carbon atom to which they are attached form a cyclopropyl ring, or
R⁴ and R⁵ together with the carbon atom to which they are attached form a cyclopropyl ring;
**R⁶** is selected from hydrogen and (1-4C)alkyl;
**A** is selected from hydrogen, a group of the formula Z-(CR⁸R⁹)ₚ- and R¹⁰,
wherein p is 1, 2, 3, or 4,
R⁸ and R⁹ are each, independently, selected from hydrogen and (1-4C)alkyl, or an R⁸ and an R⁹ group attached to the same carbon atom form a cyclopropyl ring,
Z is selected from hydrogen, OR¹¹ and NR¹²R¹³, wherein R¹¹, R¹² and R¹³ are each, independently, selected from hydrogen and (1-4C)alkyl, and
R¹⁰ is selected from (1-4C)alkoxy and NR¹²R¹³, wherein R¹² and R¹³ are as defined above,
and wherein any CH₂ or CH₃ group within a Z or an R¹⁰ group optionally bears on each said CH₂ or CH₃ group one or more substituents independently selected from halogeno, (1-4C)alkyl, hydroxy and (1-4C)alkoxy;
or a pharmaceutically acceptable salt thereof.

2. A quinazoline derivative of the Formula **I** according to claim 1, wherein R¹ is selected from hydrogen, hydroxy, methoxy, ethoxy and methoxyethoxy.

3. A quinazoline derivative of the Formula **I** according to claim 2, wherein R¹ is hydrogen.

4. A quinazoline derivative of the Formula **I** according to any one or more of claims 1 to 3, wherein G¹, G², G³, G⁴ and G⁵ are each, independently, selected from hydrogen, chloro and fluoro.

5. A quinazoline derivative of the Formula **I** according to claim 4, wherein G¹, G², G³, G⁴ and G⁵ are all hydrogen.

6. A quinazoline derivative of the Formula **I** according to any one or more of claims 1 to 5, wherein X¹ is C(R⁷)₂, wherein each R⁷ is, independently, selected from hydrogen and (1-4C)alkyl.

7. A quinazoline derivative of the Formula **I** according to claim 6, wherein X¹ is CH₂.

8. A quinazoline derivative of the Formula **I** according to any one or more of claims 1 to 7, wherein Q¹ is selected from phenyl and a 5- or 6-membered monocyclic heteroaryl ring, which ring contains 1, 2 or 3 heteroatoms independently selected from oxygen, nitrogen and sulfur, which phenyl or heteroaryl group optionally bears 1, 2 or 3 substituents independently selected from halogeno, cyano and (1-4C)alkoxy.

9. A quinazoline derivative of the Formula **I** according to claim 8, wherein Q¹ is selected from phenyl, 2-pyridyl and 1,3-thiazol-4-yl, which optionally bears 1, 2 or 3 substituents independently selected from halogeno, cyano and (1-4C)alkoxy.

10. A quinazoline derivative of the Formula **I** according to claim 9, wherein Q¹ is 2-pyridyl, which optionally bears 1 or 2 substituents independently selected from fluoro, chloro and (1-2C)alkoxy.

11. A quinazoline derivative of the Formula **I** according to claim 10, wherein Q¹ is 2-pyridyl.

12. A quinazoline derivative of the Formula **I** according to any one or more of claims 1 to 11, wherein R², R³, R⁴ and R⁵ are each, independently, selected from hydrogen and (1-2C)alkyl.

13. A quinazoline derivative of the Formula **I** according to claim 12, wherein R², R³, R⁴ and R⁵ are each, independently, selected from hydrogen and (1-2C) alkyl, wherein at least one of R², R³, R⁴ and R⁵ is (1-2C)alkyl.

14. A quinazoline derivative of the Formula **I** according to any one or more of claims 1 to 13, wherein R⁶ is methyl.

15. A quinazoline derivative of the Formula **I** according to any one or more of claims 1 to 14, wherein A is a group of the formula Z-(CR⁸R⁹)ₚ-,
wherein p is 1 or 2,
R⁸ and R⁹ are each, independently, selected from hydrogen and (1-4C)alkyl,
Z is selected from hydrogen, OR¹¹ and NR¹²R¹³, wherein R¹¹, R¹² and R¹³ are each, independently, selected from hydrogen and (1-4C)alkyl,
and wherein any CH₂ or CH₃ group within a Z group optionally bears on each said CH₂ or CH₃ group one or more substituents independently selected from halogeno, (1-2C)alkyl and hydroxy.

16. A quinazoline derivative of the Formula **I** according to claim 15, wherein A is a group of the formula Z-(CR⁸R⁹)ₚ-,
wherein p is 1 or 2,
R⁸ and R⁹ are each, independently, selected from hydrogen and (1-2C)alkyl, and
Z is hydroxy.

17. A quinazoline derivative of the Formula **I** according to claim 16, wherein A is hydroxymethyl.

18. A quinazoline derivative of the Formula **I** according to claim 1 selected from one or more of the following:
2-hydroxy-N-methyl-N-{(2R)-2-[(4-{[1-(pyridin-2-ylmethyl)-1H-indol-5-yl]amino}quinazolin-5-yl)oxy]propyl}acetamide; and
2-hydroxy-N-methyl-N-{(1R)-1-methyl-2-[(4-{[1-(pyridin-2-ylmethyl)-1H-indol-5-yl]amino}quinazolin-5-yl)oxy]ethyl}acetamide;
or a pharmaceutically acceptable salt thereof.

19. A pharmaceutical composition which comprises a quinazoline derivative of the Formula **I**, or a pharmaceutically acceptable salt thereof, according to any one or more of claims 1 to 18 in association with a pharmaceutically acceptable diluent or carrier.

20. A pharmaceutical product which comprises a quinazoline derivative of the Formula **I,** or a pharmaceutically acceptable salt thereof, according to any one or more of claims 1 to 18 and an additional anti-tumour agent for the conjoint treatment of cancer.

21. A quinazoline derivative of the Formula **I,** or a pharmaceutically acceptable salt therefore, according to any one or more of claims 1 to 18 for use as a medicament.

22. Use of a quinazoline derivative of the Formula **I,** or a pharmaceutically acceptable salt therefore, according to any one or more of claims 1 to 18 in the manufacture of a medicament for use in the production of an anti-proliferative effect in a warm-blooded animal.

23. Use of a quinazoline derivative of the Formula **I,** or a pharmaceutically acceptable salt therefore, according to any one or more of claims 1 to 18 in the manufacture of a medicament for use in the treatment of a disease or medical condition mediated alone or in part by erbB receptor tyrosine kinase.

24. Use of a quinazoline derivative of the Formula **I,** or a pharmaceutically acceptable salt therefore, according to any one or more of claims 1 to 18 in the manufacture of a medicament for use in the prevention or treatment of those tumours which are sensitive to inhibition of one or more erbB receptor tyrosine kinase that are involved in the signal transduction steps which lead to the proliferation and/or survival of tumour cells in a warm-blooded animal.

25. Use of a quinazoline derivative of the Formula **I,** or a pharmaceutically acceptable salt therefore, according to any one or more of claims 1 to 18 in the manufacture of a medicament for the treatment of cancer.

26. A process for the preparation of a quinazoline derivative of the Formula **I,** or a pharmaceutically acceptable salt therefore, according to claim 1 which comprises:
(a) the coupling, conveniently in the presence of a suitable base, of a quinazoline of the Formula **II:** 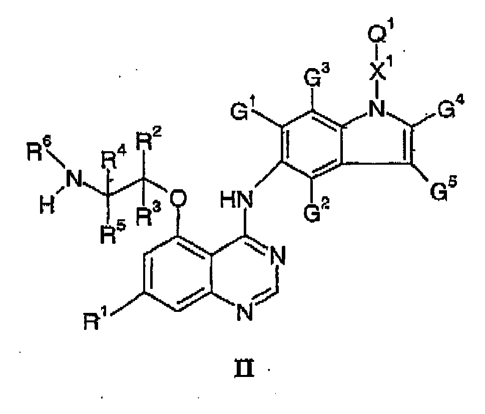 wherein R¹, R², R³, R⁴, R⁵ R⁶, X¹ Q¹ G¹, G², G³, G⁴ and G⁵ have any of the meanings defined in claim 1 except that any functional group is protected if necessary, with a carboxylic acid of the Formula **III,** or a reactive derivative thereof:
A-COOH **III**
wherein A has any of the meanings defined in claim 1 except that any functional group is protected if necessary; or
(b) for the preparation of those quinazoline derivatives of the Formula **I** wherein A is a group of the formula Z-(CR⁸R⁹)ₚ- and Z is NR¹²R¹³, the coupling of a quinazoline of the Formula **IV:** 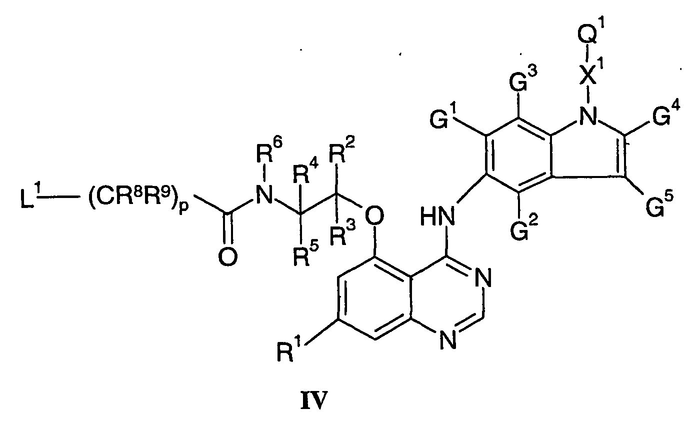 wherein L¹ is a suitable displaceable group and p, R¹, R², R³, R⁴, R⁵, R⁶, R⁸, R⁹, X¹, Q¹, G¹, G², G³, G⁴ and G⁵ have any of the meanings defined in claim 1 except that any functional group is protected if necessary, with an amine of the Formula **V:**
R¹²R¹³N-H **V**
wherein R¹² and R¹³ have any of the meanings defined in claim 1 except that any functional group is protected if necessary; or
(c) the coupling, conveniently in the presence of a suitable base, of a quinazoline of the Formula **VI:** 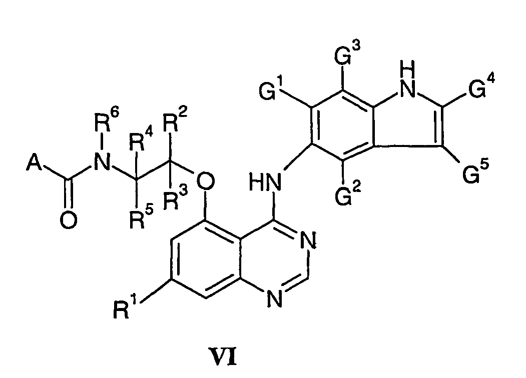 wherein R¹, R², R³, R⁴, R⁵, R⁶, A, G¹, G², G³, G⁴ and G⁵ have any of the meanings defined in claim 1 except that any functional group is protected if necessary, with a compound of the Formula **VII:**
Q¹-X¹-L² **VII**
wherein L² is a suitable displaceable group and Q¹ and X¹ have any of the meanings defined in claim 1 except that any functional group is protected if necessary;
(d) the coupling, conveniently in the presence of a suitable base, of a quinazoline of the Formula **VIII:** 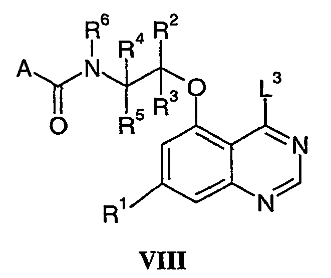 wherein L³ is a suitable displaceable group and R¹, R², R³, R⁴, R⁵, R⁶ and A have any of the meanings defined in claim 1 except that any functional group is protected if necessary, with a compound of the Formula **IX:** 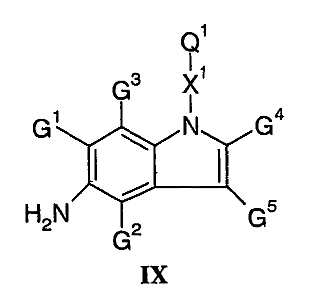 wherein G¹, G², G³, G⁴, G⁵ Q¹ and X¹ have any of the meanings defined in claim 1 except that any functional group is protected if necessary; and thereafter, if necessary:
(i) converting a quinazoline derivative of the Formula **I** into another quinazoline derivative of the Formula **I;**
(ii) removing any protecting group that is present; and/or
(iii) forming a pharmaceutically acceptable salt.

## Patentansprüche

1. Chinazolinderivate der Formel I: 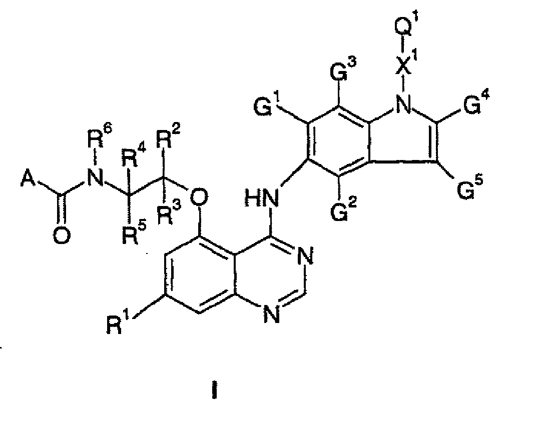 wobei:
R¹ ausgewählt ist aus Wasserstoff, Hydroxy, (1-4C)-Alkoxy und (1-4C)-Alkoxy-(1-4C)-alkoxy;
G¹, G², G³, G⁴ und G⁵ jeweils unabhängig ausgewählt sind aus Wasserstoff und Halogen;
X¹ ausgewählt ist aus SO₂, CO, SO₂N(R⁷) und C(R⁷)₂,
wobei R⁷ jeweils unabhängig ausgewählt ist aus Wasserstoff und (1-4C)-Alkyl;
Q¹ für Aryl oder Heteroaryl steht, wobei die Aryl- bzw. Heteroarylgruppe gegebenenfalls einen oder mehrere unabhängig aus Halogen, Cyano und (1-4C)-Alkoxy ausgewählte Substituenten trägt;
R², R³, R⁴ und R⁵ jeweils unabhängig ausgewählt sind aus Wasserstoff und (1-4C)-Alkyl, oder
R² und R³ zusammen mit dem Kohlenstoffatom, an das sie gebunden sind, einen Cyclopropylring bilden, oder
R⁴ und R⁵ zusammen mit dem Kohlenstoffatom, an das sie gebunden sind, einen Cyclopropylring bilden;
R⁶ ausgewählt ist aus Wasserstoff und (1-4C)-Alkyl; A ausgewählt ist aus Wasserstoff, einer Gruppe der Formel Z-(CR⁸R⁹)ₚ- und R¹⁰, wobei p für 1, 2, 3 oder 4 steht;
R⁸ und R⁹ jeweils unabhängig ausgewählt sind aus Wasserstoff und (1-4C)-Alkyl, oder eine R⁸- und eine R⁹-Gruppe, die an das gleiche Kohlenstoffatom gebunden sind, einen Cyclopropylring bilden,
Z ausgewählt ist aus Wasserstoff, OR¹¹ und NR¹²R¹³,
wobei R¹¹, R¹² und R¹³ jeweils unabhängig ausgewählt sind aus Wasserstoff und (1-4C)-Alkyl, und
R¹⁰ ausgewählt ist aus (1-4C)-Alkoxy und NR¹²R¹³,
wobei R¹² und R¹³ wie oben definiert sind,
und wobei eine CH₂- oder CH₃-Gruppe in einer Z-oder einer R¹⁰-Gruppe jeweils gegebenenfalls an jeder dieser CH₂- bzw. CH₃-Gruppen einen oder mehrere unabhängig aus Halogen, (1-4C)-Alkyl, Hydroxy und (1-4C)-Alkoxy ausgewählte Substituenten trägt;
und deren pharmazeutisch annehmbare Salze.

2. Chinazolinderivate der Formel I nach Anspruch 1, wobei R¹ ausgewählt ist aus Wasserstoff, Hydroxy, Methoxy, Ethoxy und Methoxyethoxy.

3. Chinazolinderivate der Formel I nach Anspruch 2, wobei R¹ für Wasserstoff steht.

4. Chinazolinderivate der Formel I nach einem oder mehreren der Ansprüche 1 bis 3, wobei G¹, G², G³, G⁴ und G⁵ jeweils unabhängig ausgewählt sind aus Wasserstoff, Chlor und Fluor.

5. Chinazolinderivate der Formel I nach Anspruch 4, wobei G¹, G², G³, G⁴ und G⁵ jeweils für Wasserstoff stehen.

6. Chinazolinderivate der Formel I nach einem oder mehreren der Ansprüche 1 bis 5, wobei X¹ für C(R⁷)₂ steht, wobei R⁷ jeweils unabhängig ausgewählt ist aus Wasserstoff und (1-4C)-Alkyl.

7. Chinazolinderivate der Formel I nach Anspruch 6, wobei X¹ für CH₂ steht.

8. Chinazolinderivate der Formel I nach einem oder mehreren der Ansprüche 1 bis 7, wobei Q¹ ausgewählt ist aus Phenyl und einem 5- oder 6gliedrigen monocyclischen Heteroarylring, wobei dieser Ring 1, 2 oder 3 Heteroatome, unabhängig ausgewählt aus Sauerstoff, Schwefel und Stickstoff, enthält, wobei die Phenyl- bzw. Heteroarylgruppe gegebenenfalls 1, 2 oder 3 Substituenten jeweils unabhängig ausgewählt aus Halogen, Cyano und (1-4C)-Alkoxy trägt.

9. Chinazolinderivate der Formel I nach Anspruch 8, wobei Q¹ ausgewählt ist aus Phenyl, 2-Pyridyl und 1,3-Thiazol-4-yl, welches gegebenenfalls 1, 2 oder 3 unabhängig aus Halogen, Cyano und (1-4C)-Alkoxy ausgewählte Substituenten trägt.

10. Chinazolinderivate der Formel I nach Anspruch 9, wobei Q¹ für 2-Pyridyl steht, welches gegebenenfalls 1 oder 2 unabhängig aus Fluor, Chlor und (1-2C)-Alkoxy ausgewählte Substituenten trägt.

11. Chinazolinderivate der Formel I nach Anspruch 10, wobei Q¹ für 2-Pyridyl steht.

12. Chinazolinderivate der Formel I nach einem oder mehreren der Ansprüche 1 bis 11, wobei R², R³, R⁴ und R⁵ jeweils unabhängig ausgewählt sind aus Wasserstoff und (1-2C)-Alkyl.

13. Chinazolinderivate der Formel I nach Anspruch 12, wobei R² R³, R⁴ und R⁵ jeweils unabhängig ausgewählt sind aus Wasserstoff und (1-2C)-Alkyl, wobei wenigstens einer der Reste R², R³, R⁴ und R⁵ für (1-2C)-Alkyl steht.

14. Chinazolinderivate der Formel I nach einem oder mehreren der Ansprüche 1 bis 13, wobei R⁶ für Methyl steht.

15. Chinazolinderivate der Formel I nach einem oder mehreren der Ansprüche 1 bis 14, wobei A für eine Gruppe der Formel Z-(CR⁸R⁹)ₚ- steht, wobei p für 1 oder 2 steht,
R⁸ und R⁹ jeweils unabhängig ausgewählt sind aus Wasserstoff und (1-4C)-Alkyl, Z ausgewählt ist aus Wasserstoff, OR¹¹ und NR¹²R¹³, wobei R¹¹, R¹², und R¹³ jeweils unabhängig ausgewählt sind aus Wasserstoff und (1-4C)-Alkyl, und wobei eine CH₂- oder CH₃-Gruppe in einer Z-Gruppe jeweils gegebenenfalls an jeder dieser CH₂- bzw. CH₃-Gruppen einen oder mehrere unabhängig aus Halogen, (1-2C)-Alkyl und Hydroxy ausgewählte Substituenten trägt.

16. Chinazolinderivate der Formel I nach Anspruch 15,
wobei A für eine Gruppe der Formel Z-(CR⁸R⁹)ₚ- steht,
wobei p für 1 oder 2 steht,
R⁸ und R⁹ jeweils unabhängig ausgewählt sind aus Wasserstoff und (1-2C)-Alkyl und
Z für Hydroxy steht.

17. Chinazolinderivate der Formel I nach Anspruch 16, wobei A für Hydroxymethyl steht.

18. Chinazolinderivate der Formel I nach Anspruch 1, ausgewählt aus einer oder mehreren der folgenden Verbindungen:
2-Hydroxy-N-methyl-N-{(2R)-2-[(4-{[1-(pyridin-2-ylmethyl)-1H-indol-5-yl]amino}chinazolin-5-yl)oxy]propyl}acetamid; und
2-Hydroxy-N-methyl-N-{(1R)-1-methyl-2[(4-{[1-(pyridin-2-ylmethyl)-1H-indol-5-yl]amino}-chinazolin-5-yl)oxy]ethyl}acetamid;
und deren pharmazeutisch annehmbare Salze.

19. Pharmazeutische Zusammensetzung, enthaltend ein Chinazolinderivat der Formel I oder ein pharmazeutisch annehmbares Salz davon nach einem oder mehreren der Ansprüche 1 bis 18 zusammen mit einem pharmazeutisch annehmbaren Verdünnungsmittel oder Träger.

20. Pharmazeutisches Produkt, enthaltend ein Chinazolinderivat der Formel I oder ein pharmazeutisch annehmbares Salz davon nach einem oder mehreren der Ansprüche 1 bis 18 und ein weiteres Antitumormittel zur gemeinsamen Behandlung von Krebs.

21. Chinazolinderivat der Formel I oder ein pharmazeutisch annehmbares Salz davon nach einem oder mehreren der Ansprüche 1 bis 18 zur Verwendung als Medikament.

22. Verwendung eines Chinazolinderivats der Formel I oder eines pharmazeutisch annehmbaren Salzes davon nach einem oder mehreren der Ansprüche 1 bis 18 bei der Herstellung eines Medikaments zur Verwendung beim Hervorrufen einer antiproliferativen Wirkung in einem Warmblüter.

23. Verwendung eines Chinazolinderivats der Formel I oder eines pharmazeutisch annehmbaren Salzes davon nach einem oder mehreren der Ansprüche 1 bis 18 bei der Herstellung eines Medikaments zur Verwendung bei der Behandlung einer Krankheit bzw. eines medizinischen Leidens, die/das alleine oder teilweise durch erbB-Rezeptortyrosinkinase vermittelt wird.

24. Verwendung eines Chinazolinderivats der Formel I oder eines pharmazeutisch annehmbaren Salzes davon nach einem oder mehreren der Ansprüche 1 bis 18 bei der Herstellung eines Medikaments zur Verwendung bei der Prävention oder Behandlung von Tumoren, die gegenüber einer Inhibierung einer oder mehrerer erbB-Rezeptortyrosinkinasen, die an Signalübertragungsschritten beteiligt sind, die zur Proliferation und/oder zum Überleben von Tumorzellen in einem Warmblüter führen, empfindlich sind.

25. Verwendung eines Chinazolinderivats der Formel I oder eines pharmazeutisch annehmbaren Salzes davon nach einem oder mehreren der Ansprüche 1 bis 18 bei der Herstellung eines Medikaments zur Behandlung von Krebs.

26. Verfahren zur Herstellung eines Chinazolinderivats der Formel I oder eines pharmazeutisch annehmbaren Salzes davon nach Anspruch 1, bei dem man:
(a) ein Chinazolin der Formel II: 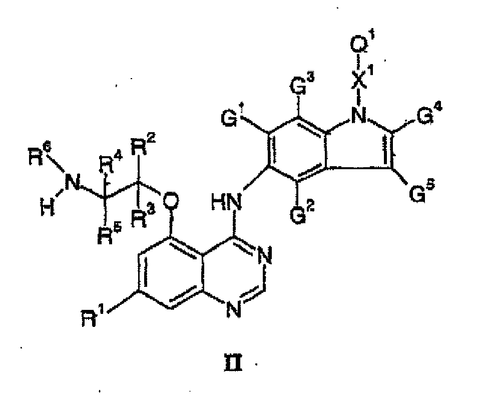 in welcher R¹, R², R³, R⁴, R⁵, R⁶, X¹, Q¹, G¹, G², G³, G⁴ und G⁵ eine der in Anspruch 1 definierten Bedeutungen haben, wobei allerdings, falls erforderlich, funktionelle Gruppen geschützt sind, zweckmäßigerweise in Gegenwart einer geeigneten Base mit einer Carbonsäure der Formel III oder einem reaktiven Derivat davon:
A-COOH **III**
in welcher A eine der in Anspruch 1 definierten Bedeutungen hat, wobei allerdings, falls erforderlich, funktionelle Gruppen geschützt sind, kuppelt; oder
(b) zur Herstellung von Chinazolinderivaten der Formel I, in welchen A für eine Gruppe der Formel Z-(CR⁸R⁹)ₚ- steht und Z für NR¹²R¹³ steht, ein Chinazolin der Formel IV: 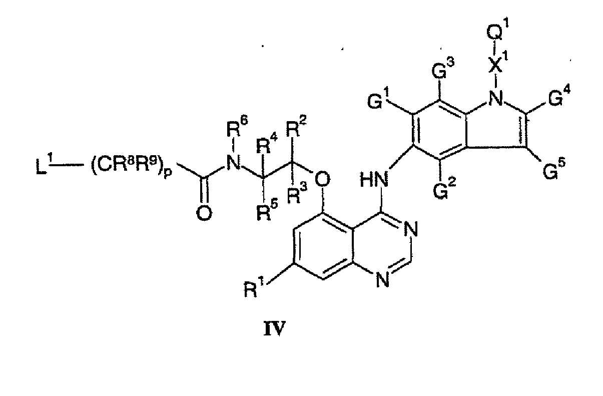 in welcher L¹ für eine geeignete Abgangsgruppe steht und p, R¹, R², R³, R⁴, R⁵, R⁶, R⁸, R⁹, X¹, Q¹, G¹, G², G³, G⁴ und G⁵ eine der in Anspruch 1 definierten Bedeutungen haben, wobei allerdings, falls erforderlich, funktionelle Gruppen geschützt sind, mit einem Amin der Formel V:
R¹²R¹³N-H **V**
in welcher R¹² und R¹³ eine der in Anspruch 1 definierten Bedeutungen haben, wobei allerdings, falls erforderlich, funktionelle Gruppen geschützt sind, kuppelt; oder
(c) ein Chinazolin der Formel VI: 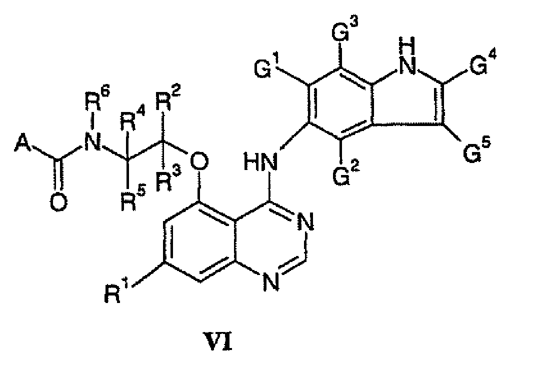 in welcher R¹, R², R³, R⁴, R⁵, R⁶, A, G¹, G², G³, G⁴ und G⁵ eine der in Anspruch 1 definierten Bedeutungen haben, wobei allerdings, falls erforderlich, funktionelle Gruppen geschützt sind, zweckmäßigerweise in Gegenwart einer geeigneten Base mit einer Verbindung der Formel VII:
Q¹-X¹-L² **VII**
in welcher L² für eine geeignete Abgangsgruppe steht und Q¹ und X¹ eine der in Anspruch 1 definierten Bedeutungen haben, wobei, allerdings, falls erforderlich, funktionelle Gruppen geschützt sind, kuppelt;
(d) ein Chinazolin der Formel VIII: 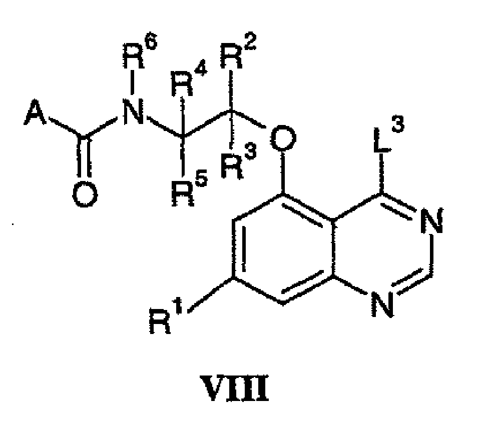 in welcher L³ für eine geeignete Abgangsgruppe steht und R¹, R², R³, R⁴, R⁵, R⁶ und A eine der in Anspruch 1 definierten Bedeutungen haben, wobei allerdings, falls erforderlich, funktionelle Gruppen geschützt sind,
zweckmäßigerweise in Gegenwart einer geeigneten Base mit einer Verbindung der Formel IX: 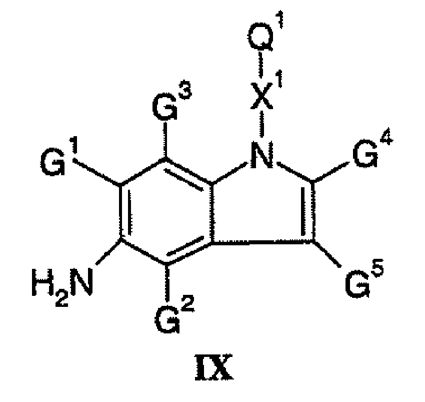 in welcher G¹, G², G³, G⁴, G⁵, Q¹ und X¹ eine der in Anspruch 1 definierten Bedeutungen haben, wobei allerdings, falls erforderlich, funktionelle Gruppen geschützt sind, kuppelt;
und anschließend, falls erforderlich:
(i) ein Chinazolinderivat der Formel I in ein anderes Chinazolinderivat der Formel I umwandelt;
(ii) eine gegebenenfalls vorhandene Schutzgruppe entfernt; und/oder
(iii) ein pharmazeutisch annehmbares Salz bildet.

## Revendications

1. Dérivé de la quinazoline de formule I : 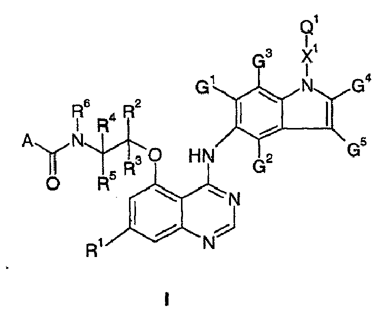 dans laquelle :
**R¹** est choisi parmi hydrogène, hydroxy, (1-4C) alcoxy et (1-4C) alcoxy (1-4C) alcoxy ;
**G¹, G², G³, G⁴ et G⁵** sont choisis chacun indépendamment parmi hydrogène et halogéno ;
**X¹** est choisi parmi SO₂, CO, SO₂N(R⁷) et C(R⁷)₂, où chaque R⁷ est choisi indépendamment parmi hydrogène et (1-4C)alkyle ;
**Q¹** est aryle ou hétéroaryle, lequel groupement aryle ou hétéroaryle porte éventuellement un ou plusieurs substituants choisis indépendamment parmi halogéno, cyano et (1-4C)alcoxy ;
**R², R³, R⁴ et R⁵** sont choisis chacun indépendamment parmi hydrogène et (1-4C)alkyle, ou
**R² et R³** ensemble avec l'atome de carbone auquel ils sont liés forment un cycle cyclopropyle, ou
**R⁴ et R⁵** ensemble avec l'atome de carbone auquel ils sont liés forment un cycle cyclopropyle ;
**R⁶** est choisi parmi hydrogène et (1-4C)alkyle ;
**A** est choisi parmi hydrogène, un groupement de formule Z-(CR⁸R⁹)ₚ- et R¹⁰,
où p est 1, 2, 3 ou 4,
**R⁸ et R⁹** sont choisis chacun indépendamment parmi hydrogène et (1-4C)alkyle, ou un groupement R⁸ et un groupement R⁹ liés au même atome de carbone forment un cycle cyclopropyle,
**Z** est choisi parmi hydrogène, OR¹¹ et NR¹²R¹³, où R¹¹, R¹² et R¹³ sont choisis chacun indépendamment parmi hydrogène et (1-4C)alkyle, et
**R¹⁰** est choisi parmi (1-4C)alcoxy et NR¹²R¹³, où R¹² et R¹³ sont tels que définis ci-dessus, et où tout groupement CH₂ ou CH₃ dans un groupement Z ou R¹⁰ porte éventuellement sur chaque dit groupement CH₂ ou CH₃ un ou plusieurs substituants choisis indépendamment parmi halogéno, (1-4C)alkyle, hydroxy et (1-4C)alcoxy ;
ou un sel pharmaceutiquement acceptable de celui-ci.

2. Dérivé de la quinazoline de formule I selon la revendication 1, **caractérisé en ce que** R¹ est choisi parmi hydrogène, hydroxy, méthoxy, éthoxy et méthoxyéthoxy.

3. Dérivé de la quinazoline de formule I selon la revendication 2, **caractérisé en ce que** R¹ est hydrogène.

4. Dérivé de la quinazoline de formule I selon une ou plusieurs des revendications 1 à 3, **caractérisé en ce que** G¹, G², G³, G⁴ et G⁵ sont choisis chacun indépendamment parmi hydrogène, chloro et fluoro.

5. Dérivé de la quinazoline de formule I selon la revendication 4, **caractérisé en ce que** G¹, G² G³, G⁴ et G⁵ sont tous hydrogène.

6. Dérivé de la quinazoline de formule I selon une ou plusieurs des revendications 1 à 5, **caractérisé en ce que** X¹ est C(R⁷)₂, où chaque R⁷ est choisi indépendamment parmi hydrogène et (1-4C)alkyle.

7. Dérivé de la quinazoline de formule I selon la revendication 6, **caractérisé en ce que** X¹ est CH₂.

8. Dérivé de la quinazoline de formule I selon une ou plusieurs des revendications 1 à 7, **caractérisé en ce que** Q¹ est choisi parmi phényle et un cycle hétéroaryle monocyclique à 5 ou 6 chaînons, lequel cycle contient 1, 2 ou 3 hétéroatomes choisis indépendamment parmi oxygène, azote et soufre, lequel groupement phényle ou hétéroaryle porte éventuellement 1, 2 ou 3 substituants choisis indépendamment parmi halogéno, cyano et (1-4C) alcoxy.

9. Dérivé de la quinazoline de formule I selon la revendication 8, **caractérisé en ce que** Q¹ est choisi parmi phényle, 2-pyridyle et 1,3-thiazol-4-yle, qui porte éventuellement 1, 2 ou 3 substituants choisis indépendamment parmi halogéno, cyano et (1-4C)alcoxy.

10. Dérivé de la quinazoline de formule I selon la revendication 9, **caractérisé en ce que** Q¹ est 2-pyridyle, qui porte éventuellement 1 ou 2 substituants choisis indépendamment parmi fluoro, chloro et (1-2C)alcoxy.

11. Dérivé de la quinazoline de formule I selon la revendication 10, **caractérisé en ce que** Q¹ est 2-pyridyle.

12. Dérivé de la quinazoline de formule I selon une ou plusieurs des revendications 1 à 11, **caractérisé en ce que** R², R³, R⁴ et R⁵ sont choisis chacun indépendamment parmi hydrogène et (1-2C)alkyle.

13. Dérivé de la quinazoline de formule I selon la revendication 12, **caractérisé en ce que** R², R³, R⁴ et R⁵ sont choisis chacun indépendamment parmi hydrogène et (1-2C)alkyle, où au moins l'un de R², R³, R⁴ et R⁵ est (1-2C)alkyle.

14. Dérivé de la quinazoline de formule I selon une ou plusieurs des revendications 1 à 13, **caractérisé en ce que** R⁶ est méthyle.

15. Dérivé de la quinazoline de formule I selon une ou plusieurs des revendications 1 à 14, **caractérisé en ce que** A est un groupement de formule Z-(CR⁸R⁹)ₚ-,
où p est 1 ou 2,
R⁸ et R⁹ sont choisis chacun indépendamment parmi hydrogène et (1-4C)alkyle,
Z est choisi parmi hydrogène, OR¹¹ et NR¹²R¹³, où R¹¹, R¹² et R¹³ sont choisis chacun indépendamment parmi hydrogène et (1-4C)alkyle,
et où tout groupement CH₂ ou CH₃ dans un groupement Z porte éventuellement sur chaque dit groupement CH₂ ou CH₃ un ou plusieurs substituants choisis indépendamment parmi halogéno, (1-2C)alkyle et hydroxy.

16. Dérivé de la quinazoline de formule I selon la revendication 15, **caractérisé en ce que** A est un groupement de formule 2-(CR⁸R⁹)ₚ-,
où p est 1 ou 2,
R⁸ et R⁹ sont choisis chacun indépendamment parmi hydrogène et (1-2C)alkyle, et
Z est hydroxy.

17. Dérivé de la quinazoline de formule I selon la revendication 16, **caractérisé en ce que** A est hydroxyméthyle.

18. Dérivé de la quinazoline de formule I selon la revendication 1, **caractérisé en ce qu'**il est choisi parmi un ou plusieurs des dérivés suivants :
le 2-hydroxy-N-méthyl-N-{(2R)-2-[(4-{[1-(pyridin-2-ylméthyl)-1H-indol-5-yl]amino}quinazolin-5-yl)oxy]propyl}acétamide ; et
le 2-hydroxy-N-méthyl-N-{(1R)-1-méthyl-2[(4-{[1-(pyridin-2-ylméthyl)-1H-indol-5-yl]amino}quinazolin-5-yl)oxy]éthyl}acétamide ;
ou un sel pharmaceutiquement acceptable de ceux-ci.

19. Composition pharmaceutique, **caractérisée en ce qu'**elle comprend un dérivé de la quinazoline de formule I, ou un sel pharmaceutiquement acceptable de celui-ci, selon une ou plusieurs des revendications 1 à 18, en association avec un diluant ou un support pharmaceutiquement acceptable.

20. Produit pharmaceutique, **caractérisé en ce qu'**il comprend un dérivé de la quinazoline de formule I, ou un sel pharmaceutiquement acceptable de celui-ci, selon une ou plusieurs des revendications 1 à 18 et un agent antitumoral supplémentaire pour le traitement conjoint du cancer.

21. Dérivé de la quinazoline de formule I, ou un sel pharmaceutiquement acceptable de celui-ci, selon une ou plusieurs des revendications 1 à 18, destiné à être utilisé comme médicament.

22. Utilisation d'un dérivé de la quinazoline de formule I, ou d'un sel pharmaceutiquement acceptable de celui-ci, selon une ou plusieurs des revendications 1 à 18, dans la fabrication d'un médicament destiné à être utilisé dans la production d'un effet anti-prolifératif chez un animal à sang chaud.

23. Utilisation d'un dérivé de la quinazoline de formule I, ou d'un sel pharmaceutiquement acceptable de celui-ci, selon une ou plusieurs des revendications 1 à 18, dans la fabrication d'un médicament destiné à être utilisé dans le traitement d'une maladie ou d'une affection médicale médiée uniquement ou en partie par le récepteur tyrosine kinase erbB.

24. Utilisation d'un dérivé de la quinazoline de formule I, ou d'un sel pharmaceutiquement acceptable de celui-ci, selon une ou plusieurs des revendications 1 à 18, dans la fabrication d'un médicament destiné à être utilisé dans la prévention ou le traitement des tumeurs qui sont sensibles à l'inhibition d'un ou plusieurs récepteurs tyrosine kinases erbB qui sont impliqués dans les étapes de transduction des signaux qui conduisent à la prolifération et/ou la survie des cellules tumorales chez un animal à sang chaud.

25. Utilisation d'un dérivé de la quinazoline de formule I, ou d'un sel pharmaceutiquement acceptable de celui-ci, selon une ou plusieurs des revendications 1 à 18, dans la fabrication d'un médicament destiné au traitement du cancer.

26. Procédé de préparation d'un dérivé de la quinazoline de formule I, ou d'un sel pharmaceutiquement acceptable de celui-ci, selon la revendication 1, **caractérisé en ce qu'**il comprend :
(a) le couplage, commodément en présence d'une base convenable, d'une quinazoline de formule II : 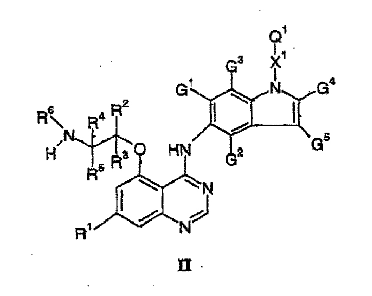 dans laquelle R¹, R², R³, R⁴, R⁵, R⁶, X¹, Q¹, G¹, G², G³ G⁴ et G⁵ ont l'une quelconque des significations définies dans la revendication 1 sauf que toute fonction est protégée si nécessaire, avec un acide carboxylique de formule III, ou un dérivé réactif de celui-ci:
A-COOH **III**
dans laquelle A a l'une quelconque des significations définies dans la revendication 1, sauf que toute fonction est protégée si nécessaire ; ou
(b) pour la préparation des dérivés de la quinazoline de formule I dans laquelle A est un groupement de formule Z-(CR⁸R⁹)ₚ- et Z est NR¹²R¹³, le couplage d'une quinazoline de formule IV : 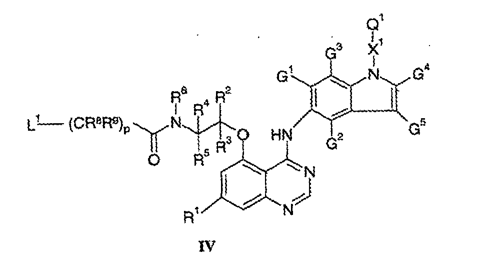 dans laquelle L¹ est un groupement déplaçable convenable et p, R¹, R², R³, R⁴, R⁵, R⁶, R⁸, R⁹, X¹, Q¹, G¹, G², G³, G⁴ et G⁵ ont l'une quelconque des significations définies dans la revendication 1 sauf que toute fonction est protégée si nécessaire, avec une amine de formule V :
R¹²R¹³N-H **V**
dans laquelle R¹² et R¹³ ont l'une quelconque des significations définies dans la revendication 1 sauf que toute fonction est protégée si nécessaire ; ou
(c) le couplage, commodément en présence d'une base convenable, d'une quinazoline de formule VI : 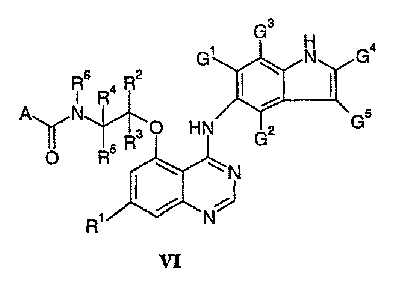 dans laquelle R¹, R², R³, R⁴, R⁵, R⁶, A, G¹, G², G³, G⁴ et G⁵ ont l'une quelconque des significations définies dans la revendication 1 sauf que toute fonction est protégée si nécessaire, avec un composé de formule VII :
Q¹-X¹L² **VII**
dans laquelle L² est un groupement déplaçable convenable et Q¹ et X¹ ont l'une quelconque des significations définies dans la revendication 1 sauf que toute fonction est protégée si nécessaire ;
(d) le couplage, commodément en présence d'une base convenable, d'une quinazoline de formule VII : 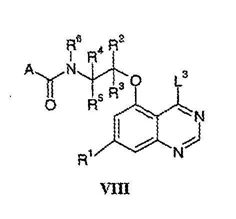 dans laquelle L³ est un groupement déplaçable convenable et R¹, R², R³, R⁴, R⁵, R⁶ et A ont l'une quelconque des significations définies dans la revendication 1 sauf que toute fonction est protégée si nécessaire, avec un composé de formule IX : 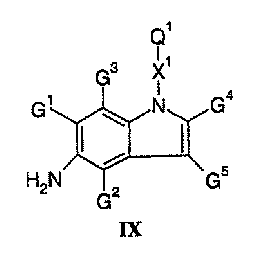 dans laquelle G¹, G², G³, G⁴, G⁵, Q¹ et X¹ ont l'une quelconque des significations définies dans la revendication 1 sauf que toute fonction est protégée si nécessaire ; et ensuite, si nécessaire ;
(i) la transformation d'un dérivé de la quinazoline de formule I en un autre dérivé de la quinazoline de formule I ;
(ii) l'élimination de tout groupement protecteur qui est présent ; et/ou
(iii) la formation d'un sel pharmaceutiquement acceptable.
